# EUROPÄISCHE PATENTSCHRIFT

(11) **EP 3 074 082 B1**
(45) Veröffentlichungstag und Bekanntmachung des Hinweises auf die Patenterteilung: **21.02.2018**
(21) Anmeldenummer: 14786672.7
(22) Anmeldetag: 21.10.2014
(51) Int. Cl.: A61B 5/155, A61M 5/142, A61M 5/158, A61M 39/02, A61M 25/00, A61B 5/145, A61B 5/15, A61B 5/153, A61B 5/00, A61M 16/06

(54) **SETZHILFE ZUM SETZEN EINES KATHETERS FÜR DIABETIKER**
INSERTION AID FOR INSERTING A CATHETER FOR DIABETICS
AUXILIAIRE DE PLACEMENT D'UN CATHÉTER POUR DIABÉTIQUE

(30) Priorität: 28.11.2013 DE 102013224431
(43) Veröffentlichungstag der Anmeldung: 05.10.2016
(73) Patentinhaber: Medizinische Universität Graz, 8036 Graz (AT)
(72) Erfinder: FRITZ, Martin, A-8723 Kobenz (AT); WÜSTER, Christian, A-9500 Villach (AT); REGITTNIG, Werner, A-8010 Graz (AT)
(74) Vertreter: Dilg, Haeusler, Schindelmann Patentanwaltsgesellschaft mbH
(86) Internationale Anmeldenummer: PCT/EP2014/072499
(87) Internationale Veröffentlichungsnummer: WO 2015/078636

(56) Entgegenhaltungen:
- EP-A1- 2 201 968
- WO-A1-2006/061354
- WO-A1-2008/065646
- US-A1- 2007 078 322
- US-A1- 2011 319 728

## Beschreibung

### Technisches Gebiet

Die vorliegende Erfindung betrifft eine Setzhilfe und ein Verfahren zum Setzen eines Katheters und eines Sensordrahts in einen Körper.

### Hintergrund der Erfindung

Patienten mit Diabetes müssen häufig, beispielsweise mehrmals täglich, den Glukosegehalt im Blut messen. Basierend darauf wird eine entsprechende Dosis an Insulin verabreicht. Dabei wird beispielsweise mit Hilfe einer Nadel die Haut penetriert und das dabei austretende Blut auf einen Messstreifen als Teil eines Messsystems aufgebracht. Nach Vorliegen des Messwertes wird die Insulindosis errechnet, wobei das Insulin mit Hilfe einer weiteren Nadel in ein Gewebe des Patienten gespritzt wird. Es sind somit mehrere Penetrationen der Haut notwendig. Um die Häufigkeit der Penetrationen und die damit verbundenen Schmerzen und Unannehmlichkeiten zu verringern, kann ein Katheter über einen längeren Zeitraum in dem Körper implantiert werden. Über diesen Katheter (sog. Verweilkatheter) kann dann ein Medikament (z.B., Insulin) verabreicht, Gewebeflüssigkeit für Glukosebestimmungen entnommen und/oder mittels eines am Katheter gelegenen Sensors die Glukose kontinuierlich gemessen werden.

US 2007/078322 A1 offenbart eine Einführungsvorrichtung für einen Sensor. Die Einführungsvorrichtung weist einen Triggerbereich auf, welcher funktional an einen Einführbereich (z.B. eine Setznadel) gekoppelt ist. Der Triggerbereich versetzt die Setznadel in Richtung Hautoberfläche eines Patienten. Ein Sensor ist mit der Setznadel derart gekoppelt, dass mittels Drückens des Triggerbereichs durch den Patienten die Setznadel zusammen mit dem Sensor nach unten in Richtung Haut gepresst wird und die Haut des Patienten penetriert wird.

US 2011/319728 A1 offenbart ein System zum Erfassen eines Parameters eines Bluts in einen Patienten, wobei das System eine vaskuläre Zugangseinrichtung mit einem inneren Fluiddurchgang zum Herstellen einer Fluidverbindung aufweist. Ein Sensor ist an der Zugangseinrichtung angeordnet und ist konfiguriert, um einen Blutparameter zu erfassen. Ein Drucksensor ist in dem inneren Fluiddurchgang der Zugangseinrichtung angeordnet. Eine Strömungssteuereinrichtung ist so konfiguriert, dass eine Fluidströmung in dem internen Fluiddurchgang in Abhängigkeit des Druckes gesteuert wird.

WO 2008/065646 A1 offenbart eine Einführungsvorrichtung zum Injizieren einer therapeutischen Flüssigkeit in einen Körper eines Patienten. Die Einführungsvorrichtung weist ein Gehäuse und darin untergebracht wenigstens eine Penetrationskartusche mit einer Nadel auf. Die Einführungsvorrichtung ist mit einem Verschiebemechanismus versehen, der bei Betätigung ein Vorschieben der Penetrationskartusche erlaubt.

WO 2006/061354 A1 offenbart eine medizinische Vorrichtung mit einer transkutanen Vorrichtungseinheit und einer Prozesseinheit. Die transkutane Vorrichtungseinheit ist angepasst, an eine Hautoberfläche eines Subjekts befestigt zu werden. Ein erstes und ein zweites Gehäuse der Vorrichtungseinheit sind derart miteinander gekoppelt, dass eine untere Fläche des zweiten Gehäuses relativ zu einer darunterliegenden Hautoberfläche bewegbar ist, während das erste Gehäuse auf der Haut aufliegt.

EP 2 201 968 A1 offenbart ein System mit einer Basiseinheit zum Stützen eines Körpers eines Patienten. Eine Einführungsvorrichtung ist an die Basiseinheit gekoppelt. Die Einführungsvorrichtung weist einen Nadelhalter auf, um eine Nadel zu halten. Ein Antriebsmechanismus bewegt den Nadelhalter linear in Einstechrichtung. Die Einführungsvorrichtung weist einen Verriegelungsmechanismus auf, um den Antriebsmechanismus zu verriegeln, wenn der Verriegelungsmechanismus in einem aktiven Zustand ist. Der Verriegelungsmechanismus entriegelt in einem inaktiven Zustand den Antriebsmechanismus, wenn die Einführungsvorrichtung mit der Basiseinheit gekoppelt ist.

### Darstellung der Erfindung

Es ist eine Aufgabe der vorliegenden Erfindung, eine einfache, bedienungsfreundliche Setzhilfe zum Setzen eines Katheters und eines Sensordrahts zu schaffen.

Diese Aufgabe wird mit einer Setzhilfe und einem Verfahren zum Setzen eines Katheters und eines Sensordrahts in einen Körper gemäß den unabhängigen Ansprüchen gelöst.

Gemäß eines ersten Aspekts der vorliegenden Erfindung wird eine Setzhilfe zum Setzen (Einführen) eines Katheters und eines Sensordrahtes in einen Körper (Organismus) geschaffen. Die Setzhilfe weist eine Setzvorrichtung und eine Austauschvorrichtung (sozusagen ein Austausch-Set bzw. ein Infusionsset) auf. Die Austauschvorrichtung weist einen Grundkörper zur Auflage auf einer Haut des Körpers, eine Setznadel und einen Sensordraht auf. Die Austauschvorrichtung ist auswechselbar bzw. austauschbar in der Setzvorrichtung angeordnet. Die Setznadel ist an den Grundkörper gekoppelt. Die Setznadel und der Katheter sind derart zueinander angeordnet, dass die Setznadel innerhalb des Katheters angeordnet ist und dass eine Spitze der Setznadel von einem proximalen Ende des Katheters entlang einer Einstechrichtung herausragt, so dass bei dem Setzen des Katheters entlang der Einstechrichtung die Spitze der Setznadel die Haut penetriert, um eine Hautöffnung zu generieren, durch welche der Katheter bis zu einer subkutanen Endposition in der Haut bzw. dem Gewebe nachführbar ist. Der Sensordraht ist mit der Setznadel derart gekoppelt, dass bei dem Setzen des Katheters entlang der Einstechrichtung der Sensordraht in der Setznadel angeordnet ist und dass bei Erreichen der subkutanen Endposition des Katheters die Setznadel entgegen der Einstechrichtung aus dem Katheter herausziehbar ist und der Sensordraht in dem Katheter verbleibt.

Der Katheter ist dabei an der Setznadel derart lösbar gekoppelt, dass bei Erreichen der subkutanen Endposition des Katheters die Setznadel entgegen der Einstechrichtung aus dem Katheter herausziehbar ist während der Katheter in der subkutanen Endposition verharrt.

Gemäß eines weiteren Aspekts der vorliegenden Erfindung wird eine Setzhilfe zum Setzen (Einführen) eines Katheters in einen Körper (Organismus) geschaffen. Die Austauschvorrichtung weist ferner in einer beispielhaften Ausführungsform einen Kolben, und einen Nadelhalter, an welchem die Setznadel befestigt ist, auf.

Der Kolben ist in dem Grundkörper verschiebbar angeordnet. Der Katheter ist derart mittels der Setznadel an den Grundkörper lösbar (direkt oder indirekt) gekoppelt, dass bei Erreichen der subkutanen Endposition des Katheters die Setznadel entgegen der Einstechrichtung aus dem Katheter herausziehbar ist und der Katheter in der subkutanen Endposition verharrt. Der Nadelhalter ist mit der Setznadel entlang der Einstechrichtung verschiebbar an dem Grundkörper angeordnet, wobei der Nadelhalter mit dem Katheter derart gekoppelt ist, dass bei dem Setzen des Katheters der Nadelhalter den Katheter entlang der Einstechrichtung vortreibt. Der Kolben ist mit dem Nadelhalter derart gekoppelt, dass mittels des Kolbens der Nadelhalter entlang der Einstechrichtung vortreibbar ist.

Gemäß eines weiteren Aspekts der vorliegenden Erfindung wird ein Verfahren zum Setzen eines Katheters und eines Sensordrahts in einen Körper mittels der oben beschriebenen Setzhilfe beschrieben. Die Austauschvorrichtung wird in der Setzvorrichtung eingesetzt bzw. angeordnet. Die Setzhilfe wird auf einer Haut des Körpers aufgelegt. Der Katheter wird mittels der Setzhilfe gesetzt. Anschließend wird die Austauschvorrichtung aus der Setzvorrichtung entfernt und ausgetauscht. Anschließend kann beispielsweise eine neue, unverbrauchte Austauschvorrichtung in dieselbe Setzvorrichtung erneut eingesetzt werden.

Gemäß einer weiteren beispielhaften Ausführungsform des Verfahrens wird ein Grundkörper auf einer Haut des Körpers aufgelegt, wobei eine Setznadel an den Grundkörper gekoppelt ist und wobei die Setznadel und ein Katheter derart zueinander angeordnet sind, dass die Setznadel innerhalb des Katheters angeordnet ist und dass eine Spitze der Setznadel von einem proximalen Ende des Katheters entlang einer Einstechrichtung herausragt. Die Haut wird mit der Spitze der Setznadel bei dem Setzen des Katheters entlang der Einstechrichtung penetriert, um eine Hautöffnung zu generieren, durch welche der Katheter bis zu einer subkutanen Endposition nachgeführt wird. Die Setznadel wird entgegen der Einstechrichtung aus dem Katheter bei Erreichen der subkutanen Endposition des Katheters herausgezogen. Gemäß einem weiteren Aspekt ist ein Nadelhalter mit der Setznadel entlang der Einstechrichtung verschiebbar an dem Grundkörper angeordnet, wobei der Nadelhalter mit dem Katheter derart gekoppelt ist, dass bei dem Setzen des Katheters der Nadelhalter den Katheter entlang der Einstechrichtung vortreibt. Ein Kolben ist mit dem Nadelhalter derart gekoppelt, dass mittels des Kolbens der Nadelhalter entlang der Einstechrichtung vortreibbar ist.

Als Katheter wird beispielsweise eine Nadel, beispielsweise aus Metall oder Kunststoff, verwendet. Der Katheter kann ferner einen dünnen Schlauch darstellen, durch welchen ein Medikament, z.B. Insulin, infundiert, Körperflüssigkeiten entnommen und/oder in welchem ein Sensordraht positioniert werden kann. In den entnommenen Flüssigkeiten oder am Setzort des Sensordrahtes können verschiedene Stoffe bzw. Werte gemessen werden, wie z.B. Glukose, Laktat, Sauerstoff, pH-Wert, Elektrolyte. Als Medikament können Insulin oder Insulinanaloga, Glukagon, Wachstumshormone zugeführt werden.

Die erfindungsgemäße Setzhilfe kann sowohl in der Humanmedizin als auch in der Tiermedizin eingesetzt werden.

Die Setznadel weist eine Nadel auf, welche z.B. aus Metall oder einem ähnlich harten Material gebildet ist. Die Setznadel kann massiv als Vollkörper ausgebildet sein oder ein Hohlprofil aufweisen. Ferner kann die Setznadel ein offenes Hohlprofil, wie beispielsweise ein U-Profil, aufweisen. Die Setznadel weist eine Spitze auf, die beispielsweise eine kleine Schneide an dem distalen Ende aufweist, mit welcher die Haut schonend penetriert werden kann. Die Setznadel fügt der Haut sozusagen einen kleinen Schnitt zu, um eine Hautöffnung zu generieren. Die Hautöffnung wird mittels des restlichen Nadelkörpers und mit dem nachgeführten Katheter gedehnt oder weiter aufgerissen. Die Setznadel weist einen kleineren Umfang als der Innendurchmesser des Katheters auf, so dass die Setznadel innerhalb des Katheters geführt werden kann.

Als Einstechrichtung wird folgend eine Richtung verstanden, entlang welcher sich die Elemente der Setzhilfe, z.B. der Katheter, die Setznadel und der Grundkörper, während des Setzens bzw. des Einführens des Katheters bewegen, zumindest bis die subkutane Endposition erreicht ist.

Die subkutane Endposition beschreibt die gewünschte Position bzw. Einstechtiefe des Katheters oder des Sensordrahtes. Die subkutane Endposition kann je nach Anwendungsfall so gewählt werden, dass ein proximales Ende des Katheters bzw. Sensordrahtes in dem Fettgewebe, dem Muskelgewebe oder innerhalb eines Blutgefäßes vorliegt.

Der Katheter weist ein proximales, d.h. hautnahes, Ende auf. Das proximale Ende des Katheters beschreibt die tiefste Stelle des Katheters im Gewebe, wenn der Katheter die subkutane Endposition erreicht hat. Von diesem proximalen Ende des Katheters steht zumindest teilweise die Spitze der Setznadel heraus, so dass während des Setzens des Katheters entlang der Einstechrichtung zunächst die Spitze der Setznadel die Haut penetriert und anschließend bei einer weiteren Bewegung entlang der Einstechrichtung der Katheter nachgeführt wird.

Der Katheter und Sensordraht sind indirekt mittels der Setznadel an den Grundkörper zumindest bis zum Erreichen der subkutanen Endposition gekoppelt. Nachdem der Katheter und Sensordraht die subkutane Endposition im Körper erreicht haben, wird die Setznadel zusammen mit dem Grundkörper von dem Katheter und Sensordraht entkoppelt und kann von dem Katheter und Sensordraht entfernt werden. Mit anderen Worten liegen nach Erreichen der subkutanen Endposition der Katheter und Sensordraht einerseits und der Grundkörper mit der Setznadel andererseits entkoppelt bzw. entkoppelbar voneinander vor.

Der Grundkörper kann beispielsweise einen hohlzylindrischen Körper bzw. eine hohlzylindrische Hülse darstellen, wobei der Katheter, der Sensordraht und die Setznadel innerhalb des Grundkörpers angeordnet sind. Die Setznadel kann räumlich fixiert an dem Grundköper angeordnet sein oder an dem Grundkörper verschieblich angeordnet sein. Wenn die Setznadel räumlich fixiert an dem Grundkörper angeordnet ist, kann der Benutzer manuell den Grundkörper zusammen mit der Setznadel, dem Sensordraht und dem Katheter in Einstechrichtung bewegen, um die Haut zu penetrieren, bis die subkutane Endposition erreicht ist. Beispielsweise kann die Austauschvorrichtung manuell oder, wie weiter unten beschrieben, selbsttätig mittels eines Federsystems in Richtung Einstechrichtung bewegt werden, um die Haut zu penetrieren.

Der Nadelhalter weist beispielsweise einen größeren Durchmesser als der Katheter bzw. der Befestigungsbereich des Katheters auf, so dass bei Verschieben des Nadelhalters entlang der Einstechrichtung der Nadelhalter auf den Befestigungsbereich des Katheters auftrifft und diesen weiter in Richtung Einstechrichtung drückt. Entsprechend kann der Nadelhalter sich entgegen der Einstechrichtung bewegen, ohne den Katheter entgegen der Einstechrichtung mitzuschieben. Der Nadelhalter kann aus demselben Material wie die Setznadel bestehen oder ein unterschiedliches Material als die Setznadel aufweisen. Beispielsweise kann die Setznadel aus einem metallischen Material bestehen, während der Nadelhalter aus einem plastisch oder elastisch verformbaren Kunststoffmaterial besteht.

Der Kolben ist eingerichtet, gegen den Nadelhalter zu drücken und diesen entlang der Einstechrichtung vorzutreiben. Der Kolben kann beispielsweise ein zylindrisches Profil aufweisen. In einer beispielhaften Ausführungsform weist der Kolben ein holzylindrisches Profil auf. Beispielsweise ist der Kolben in einer beispielhaften Ausführungsform teleskopartig in den Grundkörper ein- und ausfahrbar ausgebildet. Der Kolben kann beispielsweise durch den Benutzer während des Setzens des Katheters in den Grundkörper hineingedrückt werden. Ferner kann ein weiterer, (zum Beispiel federbasierter) Verschiebemechanismus eingesetzt werden, welcher den Kolben während des Setzens des Katheters und Sensordrahtes in den Grundkörper hineindrückt. Wenn die Setznadel verschieblich an dem Grundkörper angeordnet ist, kann ein Verschiebemechanismus eingesetzt werden. Der Benutzer setzt den Grundkörper auf der Haut auf und aktiviert den Verschiebemechanismus, so dass die Setznadel zusammen mit dem Katheter und Sensordraht in Einstechrichtung verschoben wird, bis die subkutane Endposition erreicht ist. Der Verschiebemechanismus basiert beispielsweise auf einem federbasierten Mechanismus, der folgend in exemplarischen Ausführungsbeispielen erläutert wird.

Gemäß der vorliegenden Erfindung ist der Katheter an der Setznadel derart gekoppelt, dass bei Erreichen der subkutanen Endposition des Katheters die Setznadel entgegen der Einstechrichtung aus dem Katheter herausziehbar ist und der Katheter in der subkutanen Endposition verharrt. Die Setznadel kann beispielsweise mittels einer formschlüssigen Verbindung (z.B. mit einer Haken- bzw. Schnappverbindung) an den Katheter gekoppelt sein. Die formschlüssige Verbindung wird beispielsweise selbsttätig, d.h. bei Erreichen der subkutanen Endposition, gelöst oder mittels eines Entriegelungsmechanismus, welcher z.B. von dem Benutzer betätigt werden kann, gelöst. Ferner kann die Setznadel mittels einer kraftschlüssigen Verbindung mit dem Katheter gekoppelt werden, wobei die Setznadel aufgrund von den Reibkräften mit dem Katheter gekoppelt ist. Bei dem Herausziehen der Setznadel übersteigt die entgegen der Einstechrichtung wirkende herausziehende Kraft die Reibkraft, so dass sich die Setznadel von dem Katheter löst und herausziehen lässt. Gleichzeitig ist die kraftschlüssige Verbindung zwischen dem Katheter und dem den Katheter umgebenden Gewebe größer als die herausziehende Kraft, so dass der Katheter in der subkutanen Endposition verharrt.

Der Sensordraht kann beispielsweise steif, d.h. plastisch verformbar, elastisch verformbar oder schnurähnlich verformbar bzw. biegeschlaff ausgebildet sein. Der Sensordraht bildet zum Beispiel eine oder mehrere Elektroden aus, so dass der Sensordraht Teil eines elektrochemischen Sensorsystems sein kann. Der Sensordraht kann mit einer Sensor-Ausleseeinheit gekoppelt werden, so dass In-Vivo-Messungen durchgeführt werden können. Mittels des Sensordrahts können auf Basis elektrochemischer, enzymatischer, optischer, gravimetrischer, und/oder kalorimetrischer Messprinzipien die Konzentration von Analyten in Gewebeflüssigkeiten oder Blut, zum Beispiel die Blutglukosekonzentration, gemessen werden. Ferner können beispielsweise mehrere Sensordrähte in der Setznadel eingeordnet werden und nach dem Herausziehen der Setznadel in dem Katheter verharren. Jeder der Sensordrähte bildet beispielsweise eine Sensorelektrode.

Der Sensordraht wird beispielsweise mittels einer kraftschlüssigen Verbindung in der Setznadel während des Setzens des Katheters in seiner Position gehalten. Beispielsweise kann entsprechend der Sensordraht mit einer bestimmten Druckkraft gegen die Innenfläche der Setznadel gedrückt werden, so dass eine Reibkraft den Sensordraht mit der Setznadel fixiert. Bei dem Herausziehen der Setznadel übersteigt die herausziehende Kraft die Reibkraft, welche den Sensordraht an die Setznadel fixiert, so dass die Setznadel relativ zu dem Sensordraht aus dem Körper herausgezogen werden kann und der Sensordraht in der subkutanen Endposition verharrt.

Das Austausch-Set bzw. die Austauschvorrichtung (aufweisend z.B. den Grundkörper, den Kolben, die Setznadel, den Nadelhalter, an welchem die Setznadel befestigt ist, den Katheter und den Sensordraht) ist in der Setzvorrichtung auswechselbar angeordnet.

Die Setzvorrichtung kann somit durch den Benutzer gehandhabt werden, während der Kolben und der Grundkörper geschützt im Inneren der Setzvorrichtung vorliegen. Die Austauschvorrichtung, insbesondere die Kanüle (bzw. der Katheter), der Sensordraht, die Setznadel, der Kolben und beispielsweise der Grundkörper, kann als (steriles) Einweg-Bauteil ausgebildet sein. Dies bedeutet, dass nach einem einmaligen Penetrieren der Haut durch die Setznadel und einem Einbringen des Katheters und Sensordrahtes in die subkutane Endposition, die Austauschvorrichtung (z.B. der Grundkörper und der Kolben zusammen mit der Setznadel) ausgetauscht werden können. Eine neue Austauschvorrichtung mit einer neuen Setznadel, einem neuen Sensordraht und einem neuen Katheter kann für eine erneute weitere Penetration erneut in der Setzvorrichtung angeordnet werden.

Die Setzhilfe kann manuell bedient werden, indem der Patient die Setzvorrichtung mit der Austauschvorrichtung in Richtung Haut des Körpers bewegt und die Haut penetriert. Ferner kann beispielsweise die Setzvorrichtung auf die Haut aufgesetzt werden und anschließend der Kolben von dem Patienten manuell in Richtung Haut gedrückt werden, um die Haut zu penetrieren.

Zudem kann in der Setzvorrichtung beispielsweise ein Verschiebemechanismus für das Verschieben des Kolbens, der Setznadel, des Sensordrahtes und des Katheters, wie weiter unten beschrieben, angeordnet werden. Die Setzvorrichtung mit dem Verschiebemechanismus kann somit für eine Vielzahl von Penetration bzw. für eine Vielzahl von Setzvorgängen eingesetzt werden. Die Austauschvorrichtung ist z.B. als Einweg-Vorrichtung (aufweisend z.B. unter anderem den Grundkörper und den Kolben als Einwegbauteile) ausgebildet. Somit können beispielsweise für die Setzvorrichtung teurere und exklusivere Materialien verwendet werden, während für den Kolben und den Grundkörper preiswerte Einwegmaterialien eingesetzt werden können. Somit kann eine Setzhilfe geschaffen werden, welche eine hohe Qualität bei vergleichsweise geringeren Kosten aufweist.

Mit der erfindungsgemäßen Setzhilfe wird es dem Patienten zudem ermöglicht, in einfacher, bedienungsfreundlicher Art und Weise einen Katheter zu setzen. Mittels der erfindungsgemäßen Setzhilfe penetriert zunächst eine dafür vorgesehene Setznadel hautschonend die Haut und der Katheter mit einem größeren Durchmesser als die Setznadel wird anschließend nachgeführt. Das Nachführen des Katheters und das Herausziehen der Setznadel wird mit einem einfachen Betätigungsschritt der Setzhilfe ermöglicht, so dass eine einfache Bedienung gegeben ist.

Wie weiter oben beschrieben, kann gemäß einer weiteren beispielhaften Ausführungsform der Katheter und die Setznadel derart ausgebildet sein, dass die Kopplung mittels einer kraftschlüssigen Verbindung zwischen der Setznadel und dem Katheter erzeugbar ist.

Beispielsweise kann gemäß einer weiteren beispielhaften Ausführungsform der Erfindung der Katheter einen Befestigungsbereich und einen subkutanen Bereich aufweisen. Der subkutane Bereich ist zumindest teilweise durch die Hautöffnung in die Haut bzw. das Gewebe eindringbar, wobei der Befestigungsbereich die Kopplung bzw. die form- oder kraftschlüssige Verbindung mit der Setznadel ausbildet.

Der subkutane Bereich beschreibt beispielsweise den schlauchförmigen bzw. nadelförmigen Fortsatz des Katheters, welcher in das Gewebe eingebracht wird. Der Befestigungsbereich des Katheters ist derjenige Bereich, welcher von der Hautoberfläche nach außen hervorragt. Der Befestigungsbereich weist beispielsweise teilweise eine trichterförmige Form auf. Der Befestigungsbereich kann einen deutlich größeren Durchmesser als der subkutane Bereich aufweisen. Der Befestigungsbereich dient, wie eingangs beschrieben, zur Ausbildung der kraftschlüssigen Verbindung mit der Setznadel. Ferner kann der Befestigungsbereich zur Kopplung beispielsweise mit Sensor-Ausleseeinheiten, Insulinpumpen oder anderen funktionalen Einrichtungen dienen. Der Befestigungsbereich und der subkutane Bereich können ebenfalls unterschiedliche Materialien aufweisen. So kann der subkutane Bereich beispielsweise aus einem flexiblen Schlauchmaterial bestehen, während der Befestigungsbereich aus einem harten, nicht deformierbaren Material, wie beispielsweise Hartplastik oder Metall, besteht.

In dem Befestigungsbereich kann beispielsweise eine Membran bzw. ein Klemmkörper z.B. aus einem elastischen Material, wie beispielsweise einem elastischen Kunststoff (z.B. einem Silikonpad bzw. Silikon), angeordnet werden. Elastomere Kunststoffe können sich bei Zug- und Druckbelastung elastisch verformen, finden aber danach wieder in ihre ursprüngliche, unverformte Gestalt zurück.

Beispielsweise kann die Setznadel die Membran durchstechen, so dass die Membran elastisch verformt wird und mit einer vorbestimmten Haftkraft gegen die Setznadel drückt. Somit wird die kraftschlüssige Verbindung zwischen der Setznadel und dem Katheter erzeugt. Bei dem Herausziehen der Setznadel aus dem Klemmkörper wird die vorbestimmte Haftkraft überschritten und der Katheter verharrt in der subkutanen Endposition.

Gemäß einer weiteren beispielhaften Ausführungsform weist die Setzhilfe eine erste Feder auf, welche zwischen den Grundkörper und dem Nadelhalter derart angeordnet ist, dass eine erste Federkraft der ersten Feder entlang der Einstechrichtung wirkt, um bei dem Setzen des Katheters den Nadelhalter entlang der Einstechrichtung relativ zu dem Grundkörper vorzutreiben.

Die erste Feder kann als Zugfeder oder als Druckfeder angeordnet werden. Mittels der ersten Feder kann eine Ausführung des oben beschriebenen Verschiebemechanismus bereitgestellt werden.

Gemäß einer weiteren beispielhaften Ausführungsform ist der Kolben mit dem Nadelhalter ferner derart lösbar gekoppelt, dass bei Erreichen der subkutanen Endposition der Nadelhalter von dem Kolben entkoppelbar ist und der Nadelhalter relativ zu dem Kolben entgegen der Einstechrichtung verschiebbar ist.

Somit kann der Kolben zusammen mit dem Nadelhalter entlang der Einstechrichtung bis zu der subkutanen Endposition verfahren. Der Nadelhalter kann somit linear entlang des Kolbens geführt werden. Entsprechend kann der Katheter entlang des Kolbens linear geführt werden. Insbesondere sind die Setznadel, der Katheter, der Grundkörper sowie der Kolben konzentrisch entlang einer gemeinsamen Achse zueinander angeordnet. Nach Erreichen der subkutanen Endposition kann der Kolben in seiner Position verharren, während der Nadelhalter unabhängig von dem Kolben entgegen der Einstechrichtung relativ zu dem Kolben verschiebbar ist.

In einer beispielhaften Ausführungsform ist der Kolben als Hohlprofil, beispielsweise als hohlzylindrisches Profil, ausgebildet. Somit kann der Nadelhalter zusammen mit der Nadel nach Erreichen der subkutanen Endposition entgegen der Einstechrichtung sich relativ zu dem Kolben bewegen, so dass der Nadelhalter zusammen mit der Nadel in das Innere des Kolbens hineingeschoben wird. Die Setznadel und der Katheter befinden sich vor dem Setzen innerhalb des Kolbens. Nach dem Setzen des Katheters befinden sich ausschließlich die Setznadel und beispielsweise der unten erläuterte Niederhaltestab innerhalb des Kolbens.

Der Kolben bildet somit einen Schutzbehälter für die gebrauchte Setznadel. Somit wird beispielsweise ein guter Infektionsschutz geschaffen.

Im Folgenden wird eine beispielhaft ein Verschiebemechanismus für das Verschieben der Austauschvorrichtung, z.B. u.a. des Kolbens, der Setznadel, des Sensordrahtes und des Katheters, relativ zu der Setzvorrichtung beschrieben:
Gemäß einer beispielhaften Ausführungsform weist die Setzhilfe ferner eine zweite Feder auf, welche zwischen der Setzvorrichtung und dem Nadelhalter derart angeordnet ist, dass eine zweite Federkraft der zweiten Feder entlang der Einstechrichtung wirkt, um bei dem Setzen des Katheters den Nadelhalter entlang der Einstechrichtung direkt (oder indirekt mittels z.B. des Kolbens) vorzutreiben. Die zweite Feder kann als Zugfeder oder als Druckfeder angeordnet werden.

Gemäß einer weiteren beispielhaften Ausführungsform ist die zweite Feder derart eingerichtet, dass beim Verschieben des Nadelhalters entgegen der Einstechrichtung die zweite Feder vorgespannt wird. Wird beispielsweise der Nadelhalter zusammen mit dem Kolben und/oder dem Grundkörper in die Setzvorrichtung hineingesteckt, so kann während des Einsteckvorgangs in die Setzvorrichtung automatisch und selbsttätig die zweite Feder vorgespannt werden. Somit wird in einfacher Art und Weise die Setzhilfe geladen und für den Einsatz vorbereitet.

Gemäß einer weiteren beispielhaften Ausführungsform weist die Setzvorrichtung ein Verriegelungselement und ein Auslöseelement auf. Das Verriegelungselement verriegelt den Nadelhalter in einer Initialposition, in welche die zweite Feder vorgespannt ist. Das Auslöseelement ist mit dem Verriegelungselement derart gekoppelt, dass bei Betätigen des Auslöseelements, beispielsweise durch einen Benutzer, das Verriegelungselement den Nadelhalter freigibt und der Nadelhalter mittels der zweiten Federkraft entlang der Einstechrichtung vortreibbar ist.

Das Verriegelungselement kann beispielsweise mittels eines formschlüssigen oder eines kraftschlüssigen Wirkmechanismus den Nadelhalter in der Initialposition halten. Ein Verriegelungselement mit einem formschlüssigen Wirkmechanismus weist beispielsweise entsprechende Verriegelungshaken auf, welche in entsprechende Verriegelungsnuten des Nadelhalters eingreifen. Das Auslöseelement ist beispielsweise ein Druckschalter oder ein Schieberegler, welcher von dem Benutzer betätigt und verschoben werden kann. Das Auslöseelement entkoppelt daraufhin den Nadelhalter von dem Verriegelungselement, so dass der Nadelhalter beispielsweise basierend auf der zweiten Federkraft entlang der Einstechrichtung angetrieben werden kann.

Gemäß einer weiteren beispielhaften Ausführungsform ist der Kolben in der Setzvorrichtung verschiebbar angeordnet, wobei der Kolben einerseits mit der zweiten Feder und andererseits mit dem Nadelhalter derart gekoppelt ist, dass der Kolben und der Nadelhalter mittels der zweiten Federkraft entlang der Einstechrichtung vortreibbar sind.

Gemäß einer weiteren beispielhaften Ausführungsform weist die Setzhilfe ferner eine Rückholfeder auf, welche zwischen der Setzvorrichtung (oder dem Grundkörper) und dem Nadelhalter derart angeordnet ist, dass eine Rückholkraft der Rückholfeder entgegen der Einstechrichtung wirkt, um nach dem Setzen des Katheters und Sensordrahtes den Nadelhalter entgegen der Einstechrichtung vorzutreiben, während der Katheter und Sensordraht in der subkutanen Endposition verharren.

Mittels des Einsatzes der Rückholfeder wird somit selbsttätig die Setznadel von dem Katheter und Sensordraht entkoppelt und entgegen der Einstechrichtung aus dem Körper herausgezogen. Im Zusammenspiel mit der ersten oder zweiten Feder wird somit ein selbsttätiger Setz-Mechanismus geschaffen. Der Benutzer muss lediglich beispielsweise das Auslöseelement betätigen, um die Setzhilfe zu aktivieren. Nach dem Betätigen des Auslöseelements verschiebt sich der Nadelhalter mit der Nadel zusammen mit dem Katheter und Sensordraht bis zur subkutanen Endposition. Anschließend wird der Nadelhalter aus dem Körper mittels der Rückholfeder herausgezogen, ohne dass der Benutzer hierfür weitere Schritte unternehmen muss.

Gemäß einer weiteren beispielhaften Ausführungsform ist die Rückholfeder derart eingerichtet bzw. ausgebildet, dass beim Verschieben des Nadelhalters entlang der Einstechrichtung die Rückholfeder vorgespannt wird bzw. ist. Die Rückholfeder ist beispielsweise an den Nadelhalter gekoppelt und wird somit bei Verschieben des Nadelhalters entlang der Einstechrichtung vorgespannt. Die zweite Feder und die Rückholfeder sind derart ausgelegt, dass die zweite Federkraft ausreichend groß ist, um die Setznadel mit Sensordraht und den Katheter in das Gewebe einzutreiben und zudem die Rückholfeder vorzuspannen.

Gemäß einer weiteren beispielhaften Ausführungsform weist die Setzhilfe ferner einen Niederhaltestab auf, welcher in der Setznadel verschiebbar angeordnet ist. Der Niederhaltestab ist ausgebildet, den Sensordraht kraftschlüssig gegen die Setznadel bzw. gegen die Innenfläche der Setznadel zu drücken. Der Niederhaltestab ist ferner entlang der Einstechrichtung verschiebbar an den Grundkörper derart angeordnet, dass a) bei dem Setzen (Bewegen) des Katheters entlang der Einstechrichtung die Setznadel, der Sensordraht und der Niederhaltestab bis zu der subkutanen Endposition in die Haut zusammen vortreibbar sind und dass b) bei Erreichen der subkutanen Endposition des Katheters und Sensordrahtes der Niederhaltestab entgegen der Einstechrichtung aus dem Katheter und somit dem Körper herausziehbar ist.

Der Niederhaltestab ist beispielsweise starr ausgebildet und innerhalb der Setznadel angeordnet. Der Niederhaltestab reduziert sozusagen das freie Volumen innerhalb der Setznadel, so dass der Sensordraht oder die Sensordrähte zwischen dem Niederhaltestab und der Innenfläche der Setznadel eingespannt sind. Somit bildet der Niederhaltestab die Anpresskraft des Sensordrahts an die Innenfläche der Setznadel aus. Insbesondere kann somit die Setznadel aus dem Körper herausgezogen werden, ohne dass der Niederhaltestab und der Sensordraht herausgezogen werden und diese in der subkutanen Endposition verharren. Erst nachdem die Setznadel aus dem Körper zumindest teilweise herausgezogen ist, kann in einem anschließenden Vorgang der Niederhaltestab aus dem Körper entnommen werden und allein der Sensordraht zusammen mit dem Katheter in dem Körper verbleiben.

Dieses versetzte Herausziehen der Setznadel und des Niederhaltestab kann mit der folgenden beispielhaften Ausführungsform umgesetzt werden. Gemäß einer weiteren beispielhaften Ausführungsform weist der Niederhaltestab einen Stempel (bzw. einen Klemmring) auf, welcher mittels einer kraftschlüssigen Verbindung an den Kolben befestigt ist. Der Stempel ist ferner derart in dem Kolben angeordnet, dass in einer vorbestimmten Position im Kolben der Nadelhalter entgegen der Einstechrichtung gegen den Stempel derart drückt, dass die kraftschlüssige Verbindung zwischen dem Kolben und dem Stempel überwindbar ist und der Stempel mit dem Niederhaltestab mittels des Nadelhalter entgegen der Einstechrichtung relativ zu dem Kolben verschiebbar ist.

Die vorbestimmte Position des Stempels im Kolben ist derart gewählt, dass der Nadelhalter zunächst die Setznadel aus dem Körper herauszieht und erst gegen den Stempel drückt nachdem der Nadelhalter bereits einen gewissen Weg entgegen der Einstechrichtung im Kolben verschoben wurde. Nachdem ein Kontakt zwischen dem Stempel und dem Nadelhalter erfolgt ist, drückt bzw. zieht der Nadelhalter den Stempel ebenfalls entgegen der Einstechrichtung und die Setznadel wird zusammen mit dem Niederhaltestab aus dem Körper herausgezogen.

Somit verharrt der Niederhaltestab zunächst zusammen mit dem Sensordraht in der subkutanen Endposition im Körper, während die Setznadel bereits ein Stück weit aus dem Körper herausgezogen ist. Dies hat den Vorteil, dass die Reibkraft zwischen Sensordraht und Innenfläche der Setznadel bereits stark reduziert ist, wenn die Setznadel bereits ein Stück aus dem Körper herausgezogen ist. Ohne die Setznadel besteht keine kraftschlüssige Verbindung zwischen dem Sensordraht und dem Niederhaltestab, so dass der Niederhaltestab (nach dem bereits teilweise Herausziehens der Setznadel) problemlos herausgezogen werden kann, ohne dass der Sensordraht die subkutane Endposition wesentlich verlässt.

Gemäß einer weiteren beispielhaften Ausführungsform weist die Setzhilfe einen Auflagekörper auf, welcher auf der Haut befestigbar ist. Der Grundkörper ist austauschbar an dem Auflagekörper derart befestigt, dass der Grundkörper mit einem vorbestimmten Einstechwinkel zwischen Setznadel und Hautoberfläche fixierbar ist.

Der Auflagekörper kann beispielsweise mittels einer Klebeverbindung (z.B. mittels eines Pflasters) auf der Haut befestigt werden. Der Auflagekörper weist z.B. Koppelelemente auf, welche den Grundkörper austauschbar an den Auflagekörper koppeln. Beispielsweise kann der Grundkörper mittels eines Bajonettverschlusses oder mittels anderer formschlüssigen oder kraftschlüssigen Verbindungen (z.B. Klickverbindung oder Presspassung) an dem Auflagekörper befestigt werden.

Ferner kann das Koppelelement verstellbar an dem Auflagekörper befestigt sein, so dass ein Einstechwinkel einstellbar ist. Ein Einstechwinkel definiert den Winkel zwischen der Hautoberfläche und der Setznadel. Der Einstechwinkel liegt beispielsweise zwischen 0° und 90°. Je flacher der Winkel, desto näher liegt die subkutane Endposition an der Hauptoberfläche. Je steiler der Winkel, zum Beispiel bei 90°, desto tiefer befindet sich die subkutane Endposition im Inneren der Haut bzw. des Gewebes. Durch die Wahlmöglichkeit des Einstechwinkels kann der Benutzer den Einstechwinkel an die Dicke seiner/ihrer subkutanen Fettschicht anpassen, sodass bei gegebener Katheter- und Sensordrahtlänge die proximalen Enden von Katheter und Sensordraht im Fettgewebe zu liegen kommen und nicht in das darunter liegende Muskelgewebe hinein reichen. Das Austausch-Set kann dadurch vorteilhaft mit einer einzigen Katheter- und Sensordrahtlänge realisiert werden.

Gemäß einer weiteren beispielhaften Ausführungsform weist der Auflagekörper ein Befestigungselement auf, welches eingerichtet ist, bei Vorliegen der subkutanen Endposition des Katheters den Katheter an den Auflagekörper zu befestigen.

Das Befestigungselement bildet mit dem Katheter beispielsweise eine kraftschlüssige oder eine formschlüssige Verbindung aus. Beispielsweise kann das Befestigungselement mit dem Katheter einen Bajonettverschluss ausbilden. So kann beispielsweise das Befestigungselement einen Bolzen bzw. Pin darstellen und der Katheter, insbesondere in seinem Befestigungsbereich, eine Führungsnut zur Ausbildung des Bajonettverschlusses ausbilden. Somit kann der Grundkörper zusammen mit dem Katheter in Position an dem Auflagekörper gebracht werden und anschließend, nach dem Entkoppeln des Grundkörpers von dem Katheter, der Grundkörper von dem Auflagekörper entfernt werden.

Nach Erreichen der subkutanen Endposition ist der Katheter, wie eingangs beschrieben, von dem Grundkörper entkoppelt. Entsprechend kann der Grundkörper von dem Auflagekörper entkoppelt und gelöst werden, wobei der Katheter weiter an den Auflagekörper fixiert bleibt und von dem Grundkörper getrennt werden kann.

Nachdem der Katheter und der Sensordraht in die subkutane Endposition gebracht worden sind und an dem Auflagekörper befestigt sind, kann der Katheter und Sensordraht für die weitere Verwendung eingesetzt werden. Beispielsweise kann anschließend an den Sensordraht eine Sensorausleseeinheit befestigt werden oder eine Pumpeneinheit zur Infusion eines Medikaments an den Katheter befestigt werden.

Entsprechend kann die Setzhilfe beispielsweise eine Sensor-Ausleseeinheit aufweisen, welche an den Auflagekörper derart austauschbar befestigbar ist, dass die Sensor-Ausleseeinheit mit dem Sensordraht, welcher sich in dem Katheter befindet, zum Signalaustausch gekoppelt ist. Ferner kann in einer weiteren beispielhaften Ausführungsform die Setzhilfe eine Pumpeneinheit zur Infusion eines Medikaments, zum Beispiel Insulin, aufweisen. Die Pumpeneinheit ist an dem Auflagekörper derart austauschbar befestigbar, dass die Pumpeneinheit mit dem Katheter gekoppelt ist, so dass ein Medikament von der Pumpeneinheit in den Katheter infundiert und somit zu der subkutanen Endposition befördert werden kann.

Mit der erfindungsgemäßen Setzhilfe kann der Anwender in einfacher Art und Weise, beispielsweise mittels eines einfachen Aktivierungsschritts (zum Beispiel per Knopfdruck oder manuell), einen Katheter inklusive eines Sensordrahts in das Gewebe setzen, wobei mittels der verwendeten Setznadel die Haut schonend penetriert wird. Da die Setznadel und beispielsweise der Niederhaltestab aus der Haut herausgefahren werden und in dem Kolben gelagert werden, wird gleichzeitig ein Infektionsschutz für gebrauchte Setznadel und Niederhaltestäbe geschaffen. Die Austauschvorrichtung, aufweisend beispielsweise den Kolben, die Setznadel, den Katheter, den Sensordraht und den Grundkörper, kann austauschbar in die Setzvorrichtung eingesetzt werden, so dass ein Einweg-Mechanismus mit sterilen Einwegteilen geschaffen werden kann. Der Grundkörper und der Kolben können als Einwegteile hergestellt werden und in der Setzvorrichtung selektiv austauschbar zur Anwendung befestigt werden.

Die Setzvorrichtung kann durch die Koppelung mit der sterilen die Austauschvorrichtung wiederverwendet werden, wobei die beinhalteten Komponenten, d.h. die Setzvorrichtung und die Komponenten der Austauschvorrichtung, in funktionsgemäßer Ausführung automatisch gespannt und entspannt werden.

So kann beispielsweise gemäß einem weiteren Aspekt der vorliegenden Erfindung ein Verfahren zum Setzen eines Katheters in einem Körper mit der oben beschriebenen Setzhilfe durchgeführt werden. Gemäß dem Verfahren wird das die Austauschvorrichtung in der Setzvorrichtung (auswechselbar) angeordnet. Die Setzhilfe wird auf einer Haut des Körpers aufgelegt und des Katheters mittels der Setzhilfe bzw. der Setzvorrichtung gesetzt. Anschließend wird die Austauschvorrichtung aus der Setzvorrichtung entfernt. Für einen folgenden Setzvorgang kann eine neue die Austauschvorrichtung in die Setzvorrichtung eingebracht werden und ein erneuter, oben beschriebener Setzvorgang durchgeführt werden. Die Setzvorrichtung und beispielsweise die in der Setzvorrichtung integrierte Auslösevorrichtung (z.B. die zweite Feder etc.) kann mehrmals verwendet werden.

Es wird darauf hingewiesen, dass die hier beschriebenen Ausführungsformen lediglich eine beschränkte Auswahl an möglichen Ausführungsvarianten der Erfindung darstellen. So ist es möglich, die Merkmale einzelner Ausführungsformen in geeigneter Weise miteinander zu kombinieren, so dass für den Fachmann mit den hier expliziten Ausführungsvarianten eine Vielzahl von verschiedenen Ausführungsformen als offensichtlich offenbart anzusehen sind.

### Kurze Beschreibung der Zeichnungen

Im Folgenden werden zur weiteren Erläuterung und zum besseren Verständnis der vorliegenden Erfindung Ausführungsbeispiele unter Bezugnahme auf die beigefügten Figuren näher beschrieben. Es zeigen:
Fig. 1 eine schematische Darstellung einer Setzhilfe gemäß einer beispielhaften Ausführungsform der vorliegenden Erfindung;
Fig. 2 bis Fig. 6 schematische Schnittdarstellungen einer Austauschvorrichtung einer Setzhilfe gemäß einer beispielhaften Ausführungsform der vorliegenden Erfindung, wobei in den Fig. 2 bis Fig. 6 ein Setzzyklus dargestellt wird.
Fig. 7 eine perspektivische Darstellung der Austauschvorrichtung der Setzhilfe aus Fig. 2 bis Fig. 6;
Fig. 8 bis Fig. 11 schematische Schnittdarstellungen einer Setzhilfe mit einer Setzvorrichtung und einer Austauschvorrichtung gemäß einer beispielhaften Ausführungsform der vorliegenden Erfindung, wobei in den Fig. 8 bis Fig. 11 ein Setzzyklus dargestellt wird;
Fig. 12 eine perspektivische Darstellung der Setzhilfe aus Fig. 8 bis Fig. 11;
Fig. 13 bis Fig. 15 schematische Darstellungen eines Gehäusekörpers einer Setzvorrichtung gemäß einer beispielhaften Ausführungsform der vorliegenden Erfindung;
Fig. 16 eine schematische Darstellung einer Befestigung einer Setzvorrichtung mit einem Grundkörper und einem Kolben einer Austauschvorrichtung gemäß einer beispielhaften Ausführungsform der vorliegenden Erfindung; und
Fig. 17A bis Fig. 17C schematische Darstellungen eines Auflagekörpers mit verstellbarem Katheterhalter.

### Detaillierte Beschreibung von exemplarischen Ausführungsformen

Gleiche oder ähnliche Komponenten sind in den Figuren mit gleichen Bezugsziffern versehen. Die Darstellungen in den Figuren sind schematisch und nicht maßstäblich.

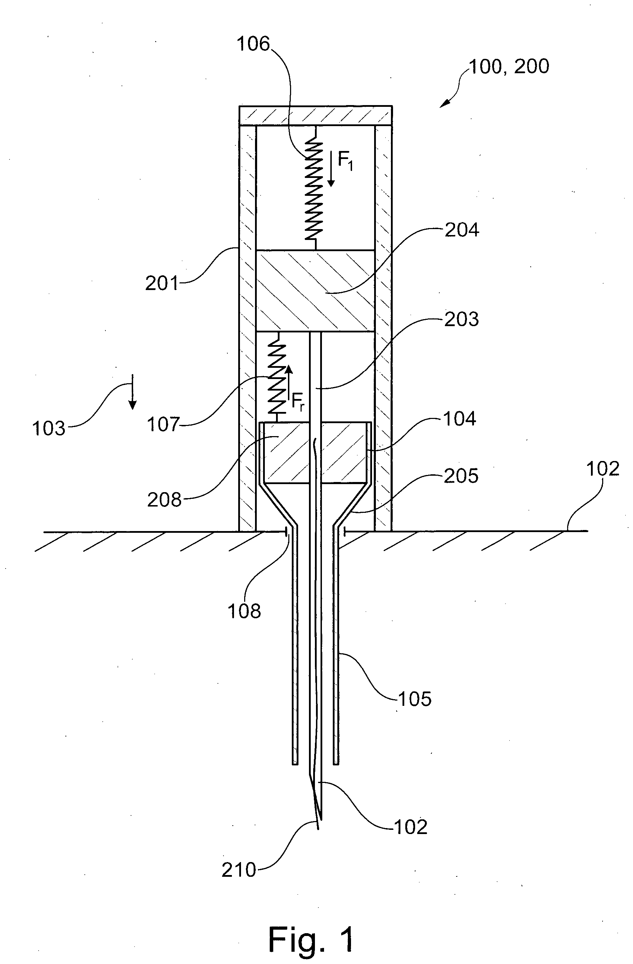

**Fig. 1** zeigt eine erste beispielhafte Ausführungsform der Setzhilfe 100. Die Setzhilfe 100 weist einen Grundkörper 201 zur Auflage auf einer Haut 101 eines Körpers (Organismus) auf. Ferner weist die Setzhilfe 100 eine Setznadel 203 und einen Katheter 205 auf. Die Setznadel 203 ist an den Grundkörper 201 gekoppelt. Die Setznadel 203 und der Katheter 205 sind derart zueinander angeordnet, dass die Setznadel 203 innerhalb des Katheters 205 angeordnet ist und dass eine Spitze 102 der Setznadel 203 von einem proximalen Ende des Katheters 104 entlang einer Einstechrichtung 103 herausragt, so dass bei dem Setzen des Katheters 205 entlang der Einstechrichtung 103 die Spitze 102 der Setznadel 203 die Haut 101 penetrieren, um eine Hautöffnung 108 zu generieren, durch welche der Katheter 205 bis zu einer subkutanen Endposition nachfolgt. Der Katheter 205 ist an der Setznadel 203 bzw. mittels der Setznadel 203 (indirekt) an den Grundkörper 201 derart gekoppelt, dass bei Erreichen der subkutanen Endposition des Katheters 205 die Setznadel 203 entgegen der Einstechrichtung 103 aus dem Katheter 205 herausziehbar ist und der Katheter 103 in der subkutanen Endposition verharrt.

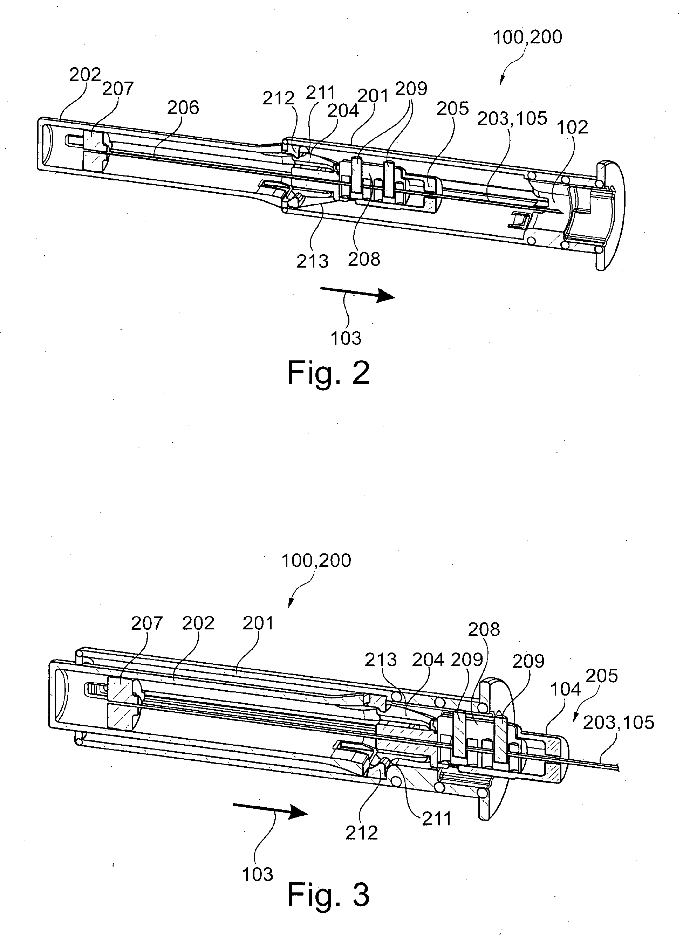

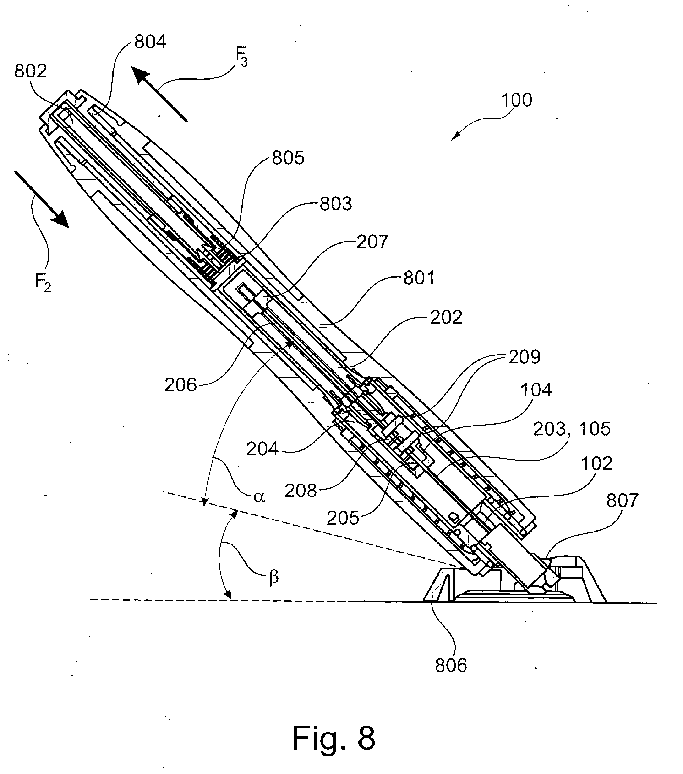

Insbesondere zeigt Fig. 1 eine Austauschvorrichtung 200, bzw. ein Infusions-Set bzw. Austausch-Set, aufweisend z.B. den Grundkörper 201, den Kolben 202 (siehe Fig. 2), die Setznadel 203, einen Nadelhalter 204, an welchem die Setznadel 203 befestigt ist, und dem Katheter 205. Die Austauschvorrichtung 200 ist z.B. in einer Setzvorrichtung 800 (siehe Fig. 8) auswechselbar angeordnet.

Die Setznadel 203 weist eine Nadel auf, welche z.B. aus Metall oder einem ähnlich harten Material gebildet ist. Die Setznadel 203 fügt mit der Spitze 102 der Haut einen kleinen Schnitt zu, um die Hautöffnung 108 zu generieren. Die Hautöffnung 108 wird mittels des restlichen Nadelkörpers und mit dem nachgeführten Katheter 205 gedehnt oder weiter aufgerissen. Die Setznadel 203 ist innerhalb des Katheters 114 geführt.

In Fig. 1 ist die Setznadel 203 und der Katheter 205 in einer subkutanen Endposition, d.h. in einer gewünschten Tiefe in der Haut 101 bzw. in einem Gewebe 109, gezeigt. Die subkutane Endposition beschreibt die gewünschte Position eines proximalen Endes des Katheters 205 bzw. Einstechtiefe des Katheters 205. Die subkutane Endposition kann je nach Anwendungsfall so gewählt werden, dass ein proximales Ende des Katheters 205 in dem Fettgewebe, dem Muskelgewebe oder innerhalb eines Blutgefäßes vorliegt.

Der Grundkörper 201 stellt, wie in Fig. 1 dargestellt, einen hohlzylindrischen Körper bzw. eine hohlzylindrische Hülse dar, wobei der Katheter 205 und die Setznadel 203 innerhalb des Grundkörpers 201 angeordnet sind. Die Setznadel 203 kann räumlich fixiert an dem Grundköper 201 angeordnet sein oder an dem Grundkörper 201 verschiebblich angeordnet sein.

Wenn die Setznadel 203 räumlich fixiert an dem Grundkörper 201 angeordnet ist, kann der Benutzer manuell den Grundkörper 201 zusammen mit der Setznadel 203 und dem Katheter 205 in Einstechrichtung bewegen, um die Haut zu penetrieren, bis die subkutane Endposition erreicht ist.

Wenn die Setznadel 203 verschieblich an dem Grundkörper 201 angeordnet ist, kann ein Verschiebemechanismus eingesetzt werden. Der Benutzer setzt den Grundkörper 201 auf der Haut 101 auf und aktiviert den Verschiebemechanismus, so dass die Setznadel 203 zusammen mit dem Katheter 205 in Einstechrichtung 103 verschoben wird bis die subkutane Endposition erreicht ist (siehe Fig. 1). Der Verschiebemechanismus basiert beispielsweise auf einem federbasierten Mechanismus. In Fig. 1 weist die Setznadel 203 einen Nadelhalter 204 auf. Der Nadelhalter 204 ist innerhalb des Grundkörpers 201 verschieblich gelagert. Ferner ist eine erste Feder 106 zwischen dem Nadelhalter 204 und dem Grundkörper 201 angeordnet. Die erste Feder 106 ist derart eingerichtet, dass eine erste Federkraft F1 in Einstechrichtung 103 werden. Mittels der ersten Federkraft F1 wird der Nadelhalter 204 und der Katheter 205 entlang der Einstechrichtung 103 verschoben, bis die subkutane Endposition des Katheters 205 erreicht ist. Die erste Feder F1 kann beispielsweise zu Beginn vorgespannt werden und mittels eines Arretierungsmechanismus in der vorgespannten Position gehalten werden. Der Benutzer kann den Verschiebemechanismus aktivieren, indem er die erste Feder 106 freigibt, so dass der Setzvorgang gestartet wird.

Alternativ oder zusätzlich zu der ersten Feder 106 kann eine weiter unten in Fig. 8 beschriebene zweite Feder 803 zwischen der Setzvorrichtung 800 der Setzhilfe 100 und dem Kolben 202 der Austauschvorrichtung 800 derart angeordnet sein, dass eine zweite Federkraft F2 der zweiten Feder 803 entlang der Einstechrichtung 103 wirkt, um bei dem Setzen des Katheters 205 den Nadelhalter 204 entlang der Einstechrichtung 103 direkt (oder indirekt mittels z.B. des Kolbens 202) vorzutreiben.

Der Katheter 205 ist an der Setznadel 203 derart gekoppelt, dass bei Erreichen der subkutanen Endposition des Katheters 205 die Setznadel 203 entgegen der Einstechrichtung 103 aus dem Katheter 205 herausziehbar ist und der Katheter 205 in der subkutanen Endposition verharrt. Die Setznadel 203 kann beispielsweise mittels einer formschlüssigen Verbindung (z.B. mit einer Haken- bzw. Schnappverbindung) an den Katheter 205 gekoppelt sein. Die formschlüssige Verbindung wird beispielsweise selbsttätig, d.h. bei Erreichen der subkutanen Endposition, gelöst oder mittelst eines Entriegelungsmechanismus, welcher von dem Benutzer betätigt werden kann, gelöst. Ferner kann die Setznadel 203 mittels einer kraftschlüssigen Verbindung mit dem Katheter 205 gekoppelt werden, wobei die Setznadel 203 aufgrund von den Reibkräften mit dem Katheter 205 gekoppelt ist. Bei dem Herausziehen der Setznadel 203 übersteigt die entgegen der Einstechrichtung 103 wirkende herausziehende Kraft die Reibkraft, so dass sich die Setznadel 203 von dem Katheter 205 löst und herausziehen lässt. Gleichzeitig ist die kraftschlüssige Verbindung zwischen dem Katheter 205 und dem den Katheter 205 umgebenden Gewebe größer als die herausziehende Kraft, so dass der Katheter 205 in der subkutanen Endposition verharrt.

Das Herausziehen der Setznadel 203 aus dem Katheter 205 nach dem Erreichen der subkutanen Endposition kann manuell oder federbasiert erfolgen. Wie in Fig. 1 dargestellt kann zwischen dem Nadelhalter 204 und einem Bereich des Grundkörpers 201 eine Rückholfeder 107 angeordnet werden. Eine Federkraft Fr der Rückholfeder 107 drückt den Nadelhalter 204 entgegen der Einstechrichtung 103. Ein weiterer Mechanismus kann die Rückholfeder 107 nach Erreichen der subkutanen Endposition des Katheters 205 freigeben, so dass der Nadelhalter 204 nach dem Erreichen der subkutanen Endposition zusammen mit der Setznadel 203 aus dem Gewebe entgegen der Einstechrichtung 103 herausgezogen wird.

Der Katheter 205 weist einen Befestigungsbereich 104, mittels welchem der Katheter 205 an der Setznadel 203 befestigbar ist, und einen subkutanen Bereich 105 auf. Der subkutane Bereich 105 ist zumindest teilweise durch die Hautöffnung 108 in die Haut 101 bzw. Gewebe 109 eindringbar, wobei der Befestigungsbereich 104 eine (kraftschlüssige oder formschlüssige) Verbindung mit der Setznadel 203 ausbildet.

Der Befestigungsbereich 104 kann beispielsweise eine Membran bzw. einen Klemmkörper 208 aus einem elastischen Material, wie beispielsweise einem elastischen Kunststoff (z.B. einem Silikonpad bzw. Silikon), aufweisen. Beispielsweise kann die Setznadel 203 den Klemmkörper 208 durchstechen, so dass der Klemmkörper 208 elastisch verformt wird und mit einer vorbestimmten Haftkraft gegen die Setznadel 203 drückt. Somit wird die kraftschlüssige Verbindung zwischen der Setznadel 203 und dem Katheter 205 erzeugt. Bei dem Herausziehen der Setznadel 203 aus dem Katheter 205 wird die vorbestimmte Haftkraft überschritten und die Setznadel 203 wird herausgezogen und der Katheter 205 verharrt in seiner Position.

Ferner kann der Befestigungsbereich 104 zur Kopplung beispielsweise mit einem Auflagekörper 806 (siehe Fig. 8), Sensor-Ausleseeinheiten, Insulinpumpen oder anderen funktionalen Einrichtungen dienen.

In der Setznadel 203 kann ferner ein Sensordraht 210 angeordnet sein. Der Sensordraht 210 ist mit der Setznadel 203 derart gekoppelt, dass bei dem Setzen des Katheters 205 entlang der Einstechrichtung der Sensordraht 210 in der Setznadel 203 angeordnet ist und dass bei Erreichen der subkutanen Endposition des Katheters 205 die Setznadel 203 entgegen der Einstechrichtung 103 aus dem Katheter 205 herausziehbar ist und der Sensordraht 210 in dem Katheter 205 verbleibt.

Der Sensordraht 210 bildet zum Beispiel eine Elektrode aus. Der Sensordraht 210 wird beispielsweise mittels einer kraftschlüssigen Verbindung in der Setznadel 203 während des Setzens des Katheters 205 in seiner Position relativ zu der Setznadel 203 gehalten. Beispielsweise kann entsprechend der Sensordraht 210 mit einer bestimmten Druckkraft gegen die Innenfläche der Setznadel 203 gedrückt werden, so dass eine Reibkraft den Sensordraht 210 mit der Setznadel 203 fixiert. Bei dem Herausziehen der Setznadel 203 übersteigt die herausziehen Kraft die Reibkraft, welche den Sensordraht 210 an die Setznadel 203 fixiert, so dass die Setznadel 203 relativ zu dem Sensordraht 210 aus dem Körper herausgezogen werden kann und der Sensordraht 210 in der subkutanen Endposition verharrt.

Nach dem Setzen des Katheters 205 und Sensordrahtes 210 kann sich der Nadelhalter 204 zusammen mit der Setznadel 203 komplett in das Innenvolumen des Grundkörpers 201 zurückziehen und ist somit von der Umgebung des Grundkörpers 201 isoliert. Somit wird ein Infektrisiko von gebrauchten Setznadel 203 reduziert.

**Fig. 2** bis **Fig. 6** zeigen perspektivische Schnittdarstellungen der Austauschvorrichtung 200 einer Setzhilfe 100 gemäß einer beispielhaften Ausführungsform der vorliegenden Erfindung, wobei in den Fig. 2 bis Fig. 6 ein Setzzyklus des Katheters 205 und eines Sensordrahtes 210 dargestellt wird.

Die Setzhilfe 100 in Fig. 2 bis Fig. 6 weist im Wesentlichen dieselben Merkmale wie die Setzhilfe 100 aus Fig. 1 auf. Zusätzlich weist die Setzhilfe 100 einen Kolben 202 auf, welcher teleskopartig in den Grundkörper 201 ein- und ausfahrbar angeordnet ist. Der Nadelhalter 204 ist derart an den Kolben 202 gekoppelt, dass bei Bewegung des Kolbens 202 der Nadelhalter 204 zusammen mit der Setznadel 203 und dem Katheter 205 entlang der Einstechrichtung 103 vortreibbar ist bis die subkutane Endposition erreicht ist (siehe Fig. 3).

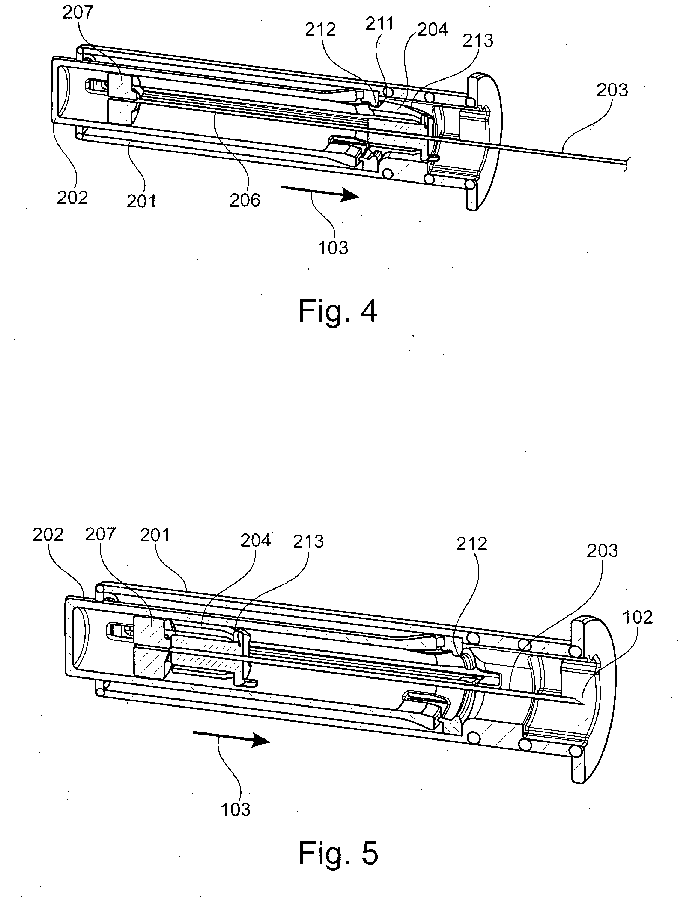

Nach dem Erreichen der subkutanen Endposition kann der Kolben 202 und der Nadelhalter 204 voneinander gelöst werden und der Nadelhalter 204 verschiebt sich relativ zu dem Kolben 202 entgegen der Einstechrichtung 103. Der Nadelhalter 204 verschiebt sich somit in das Innenvolumen des Kolbens 202 und wird darin nach dem Setzen des Katheters 205 verstaut (siehe Fig. 5).

Das Entkoppeln des Nadelhalters 204 von dem Kolben 202 kann beispielsweise mittels einer formschlüssigen (z.B. Schnapp- oder Klick-) Verbindung bereitgestellt werden. Wie in Fig. 2 bis Fig. 6 dargestellt, kann der Nadelhalter 204 eine Kerbe 211 aufweisen und der Kolben 202 kann einen Schnapphaken 212 aufweisen. Der Nadelhalter 204 kann elastisch verformbar ausgebildet sein, so dass dieser sich an einer gewünschten Position zusammendrücken lässt, um die Verbindung zwischen der Kerbe 211 und dem Schnapphaken 212 zu lösen. Beispielsweise kann der Nadelhalter 204 eine keilförmige Fläche 213 aufweisen, welche bei Erreichen der subkutanen Endposition des Katheters 205 gegen einen Anschlag oder eine weitere keilförmige Fläche des Grundkörpers 201 geschoben werden. Die weitere keilförmige Fläche kann beispielsweise an einer Innenseite des Grundkörpers 201 entsprechend ausgebildet werden. Bei Verschieben des Nadelhalters 204 entlang der keilförmigen Fläche wird der Nadelhalter 204 im Bereich seiner keilförmigen Fläche 213 zusammengedrückt, so dass bei dem Erreichen des subkutanen Endposition des Katheters 205 der Nadelhalter 204 von dem Schnapphaken 212 entkoppelt wird und sich der Nadelhalter 204 in das Innenvolumen des Kolben 202 verschiebt. Das Zurückschieben des Nadelhalters 204 entgegen der Einstechrichtung 103 nach dem Erreichen der subkutanen Endposition des Katheters 205 kann beispielsweise mittels der Rückholfeder 107 durchgeführt werden.

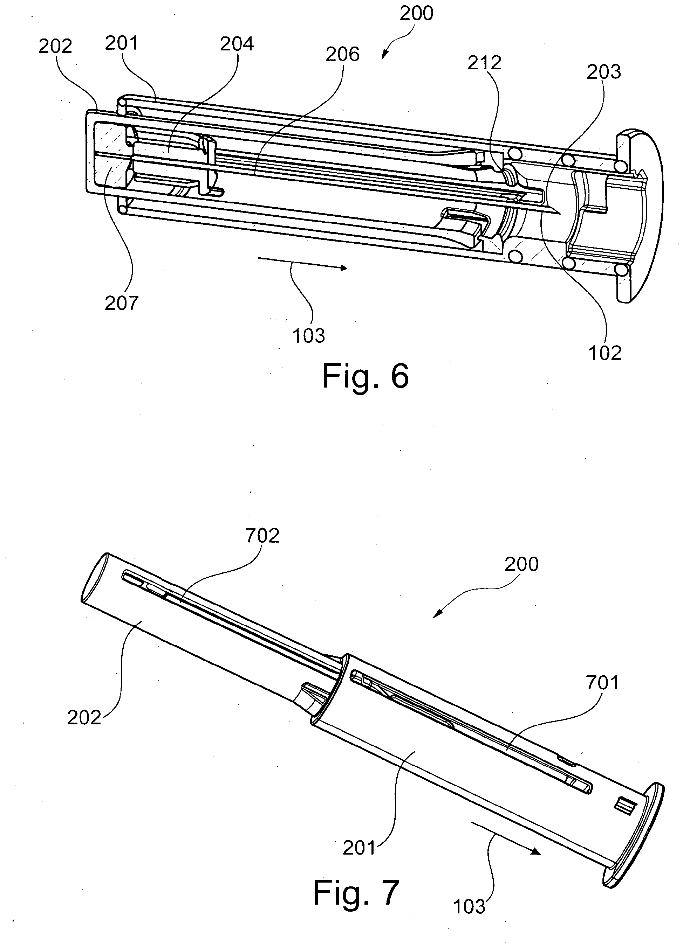

Ferner wird in der Setzhilfe 100 in Fig. 2 bis Fig. 6 ein Niederhaltestab 206 dargestellt. Der Niederhaltestab 206 ist in der Setznadel 203 verschiebbar angeordnet ist. Der Niederhaltestab 206 ist ausgebildet, den Sensordraht 210 kraftschlüssig gegen die Setznadel 203 bzw. gegen die Innenfläche der Setznadel 203 zu drücken. Der Niederhaltestab 206 ist ferner entlang der Einstechrichtung 103 verschiebbar an den Grundkörper 201 derart angeordnet, dass bei dem Setzen (Bewegen) des Katheters entlang der Einstechrichtung 103 die Setznadel 203, der Sensordraht 210 und der Niederhaltestab 206 bis zu der subkutanen Endposition in dem Gewebe 109 zusammen vortreibbar sind (siehe Fig. 2, Fig. 3) und dass bei Erreichen der subkutanen Endposition des Katheters 205 der Niederhaltestab 206 entgegen der Einstechrichtung 103 aus dem Katheter 205 herausziehbar ist (siehe Fig. 5, Fig. 6).

Der Niederhaltestab 206 reduziert das freie Volumen innerhalb der Setznadel 203, so dass der Sensordraht 210 zwischen dem Niederhaltestab 206 und der Innenfläche der Setznadel 203 eingespannt ist. Somit bildet der Niederhaltestab 206 die Anpresskraft des Sensordrahts 113 an die Innenfläche der Setznadel 203 aus. Insbesondere kann somit die Setznadel 203 aus dem Körper herausgezogen werden, ohne dass der Niederhaltestab 206 und der Sensordraht 210 herausgezogen werden und diese in der subkutanen Endposition verharren (vgl. Fig. 4 mit Fig. 5). Erst nachdem die Setznadel 203 aus dem Körper zumindest teilweise herausgezogen ist, kann in einem anschließenden Vorgang der Niederhaltestab 206 aus dem Körper entnommen werden und allein der Sensordraht 210 zusammen mit dem Katheter 205 in dem Körper verbleiben.

Dieses versetzte Herausziehen der Setznadel 203 und des Niederhaltestab 206 wird erzielt, indem ein Stempel 207 des Niederhaltestabs 206 eine kraftschlüssige Verbindung mit dem Kolben 202 ausbildet. Der Stempel 207 ist derart in dem Kolben 202 angeordnet, dass in einer vorbestimmten Position im Kolben 202 der Nadelhalter 204 entgegen der Einstechrichtung 103 gegen den Stempel 207 derart drückt, dass die kraftschlüssige Verbindung zwischen dem Kolben 202 und dem Stempel 207 überwunden wird und der Stempel 207 mit dem Niederhaltestab 206 mittels des Nadelhalters 204 entgegen der Einstechrichtung 103 relativ zu dem Kolben 202 verschoben wird (siehe Fig. 5, Fig. 6).

Die vorbestimmte Position des Stempels 207 im Kolben 202 ist derart gewählt, dass der Nadelhalter 204 zunächst die Setznadel 203 aus dem Körper heraus zieht und erst dann gegen den Stempel 207 drückt, nachdem der Nadelhalter 204 bereits einen gewissen Weg entgegen der Einstechrichtung 103 im Kolben 202 verschoben wurde. Somit verharrt der Niederhaltestab 206 zunächst zusammen mit dem Sensordraht 210 in der subkutanen Endposition im Körper, während die Setznadel 203 bereits ein Stück weit aus dem Körper herausgezogen ist.

Ferner wird in Fig. 2 und Fig. 3 der Katheter 205 zusammen mit dem Befestigungsbereich 104 und dem subkutanen Bereich 105 dargestellt. In dem Befestigungsbereich 104 ist der Klemmkörper 208 angeordnet. Ferner weist der Klemmkörper 208 zumindest eine Kontaktfläche 209 auf. Die Kontaktfläche 209 ist beispielsweise selbst elastisch verformbar oder in dem elastisch verformbaren Klemmkörper 208 befestigt. Die Setznadel 203, welche den innenliegenden Sensordraht 210 enthält, durchsticht den elastisch verformbaren Klemmkörper 208. Nachdem die Setznadel 203 aus dem elastisch verformbaren Klemmkörper 208 und entsprechend dem Katheter 205 herausgezogen ist, schließt der elastisch verformbare Klemmkörper 208 das Loch, durch welches die Setznadel 203 hindurchgesteckt wurde. In diesem Loch befindet sich nach dem Herausziehen der Setznadel 203 weiterhin der Sensordraht 210 und die Kontaktfläche 209 drückt auf den Sensordraht 210, sodass ein leitender Kontakt zwischen Sensordraht 210 und den Kontaktflächen 209 erzeugt wird. Zudem können die Kontaktflächen 209 mit einer Sensor-Ausleseeinheit verbunden werden, sodass Messsignale des Sensordrahts 113 ausgelesen werden können.

Fig. 7 zeigt eine perspektivische Darstellung der Setzhilfe 100 aus Fig. 2 bis Fig. 6. Die Setzhilfe 100 zeigt den Kolben 202 und den Grundkörper 201 in einer Initialposition. Der Grundkörper 201 weist einen ersten Spalt/Nut 701 auf, welcher sich entlang der Einstechrichtung 103 erstreckt. In diesem ersten Spalt 701 kann beispielsweise der Kolben 202 (z.B. mittels in dem ersten Spalt 701 geführte Führungsbolzen des Kolbens 202) verschiebbar geführt werden. Der Kolben 202 weist beispielsweise einen zweiten Spalt 702 auf, welcher sich entlang der Einstechrichtung 103 erstreckt. In diesem zweiten Spalt 702 kann beispielsweise der Nadelhalter 204 (z.B. mittels in dem zweiten Spalt 702 geführte weitere Führungsbolzen des Nadelhalters 204) verschiebbar geführt werden.

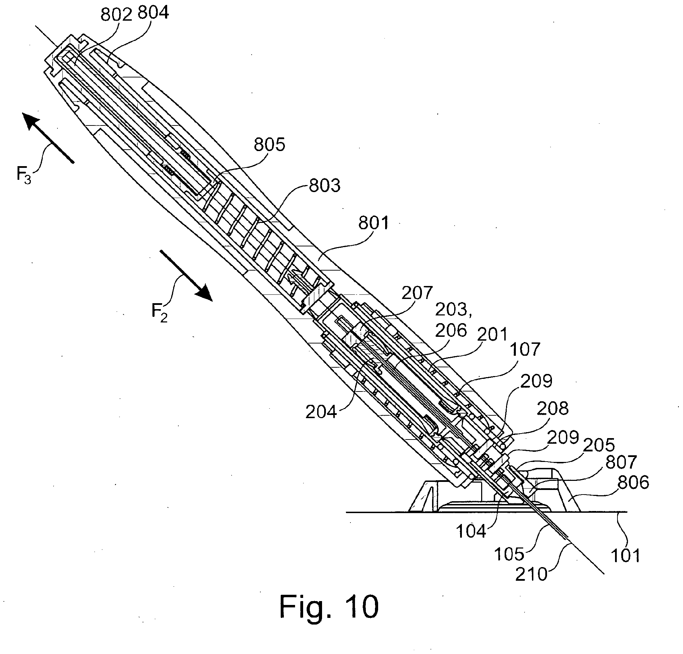

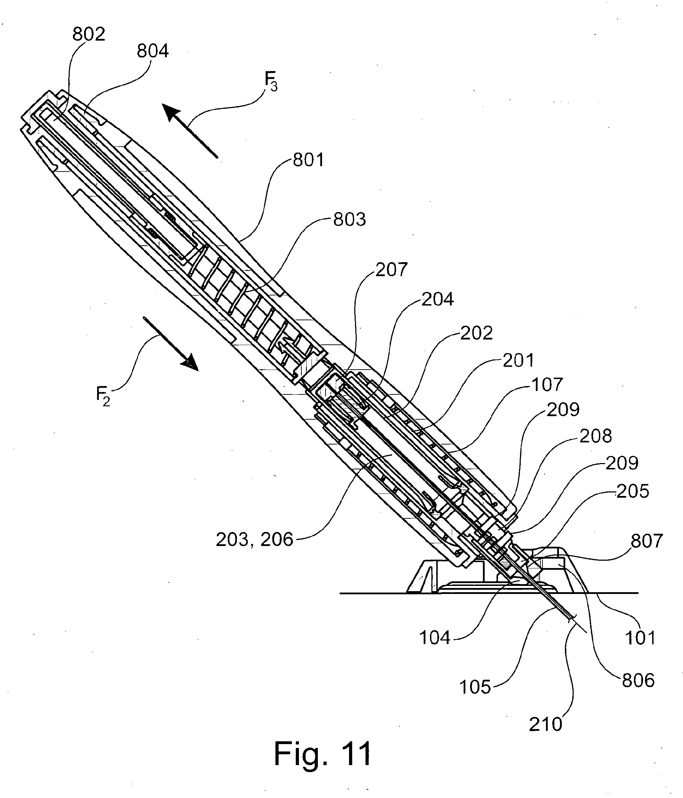

**Fig. 8** bis **Fig. 11** zeigen perspektivische Schnittdarstellungen einer Setzhilfe 100 mit einer Setzvorrichtung 800 gemäß einer beispielhaften Ausführungsform der vorliegenden Erfindung, wobei in den Fig. 8 bis Fig. 11 ein Setzzyklus des Katheters 205 und Sensordrahtes 210 dargestellt wird.

Die Setzhilfe 100 in Fig. 8 bis Fig. 11 weist im Wesentlichen dieselben Merkmale wie die Setzhilfe 100 aus Fig. 2 bis Fig. 7 auf. Die Austauschvorrichtung 200 weist den Grundkörper 201, den Kolben 202, die Setznadel 203, den Nadelhalter 204, an welchem die Setznadel 203 befestigt ist, den Sensordraht 210 und den Katheter 205 auf, wobei die Austauschvorrichtung 200 in der Setzvorrichtung 800 austauschbar angeordnet ist.

Insbesondere sind der Kolben 202 und der Grundkörper 201 in der Setzvorrichtung 800 austauschbar einsetzbar. Die Setzhilfe 100 besteht beispielsweise aus einem hohlzylindrischen Gehäuse.

Die Setzhilfe 100 weist beispielsweise einen Verschiebemechanismus für das Verschieben des Kolbens 202, der Setznadel 203 und des Katheters 205 auf. Eine zweite Feder 803 ist zwischen der Setzvorrichtung 800 und dem Kolben 202 der Austauschvorrichtung 800 derart angeordnet ist, dass eine zweite Federkraft F2 der zweiten Feder 803 entlang der Einstechrichtung 103 wirkt, um bei dem Setzen des Katheters 205 den Nadelhalter 204 entlang der Einstechrichtung 103 direkt (oder indirekt mittels z.B. des Kolbens 202) vorzutreiben.

Beim Verschieben des Nadelhalters 204 entgegen der Einstechrichtung 103 wird die zweite Feder 803 vorgespannt. Wird beispielsweise der Nadelhalter 204 zusammen mit dem Kolben 202 und/oder dem Grundkörper 201 in die Setzvorrichtung 800 hineingesteckt, so kann während des Einsteckvorgangs in die Setzvorrichtung 800 automatisch und selbsttätig die zweite Feder 803 vorgespannt werden. Somit wird in einfacher Art und Weise die Setzhilfe 100 geladen und für den Einsatz vorbereitet.

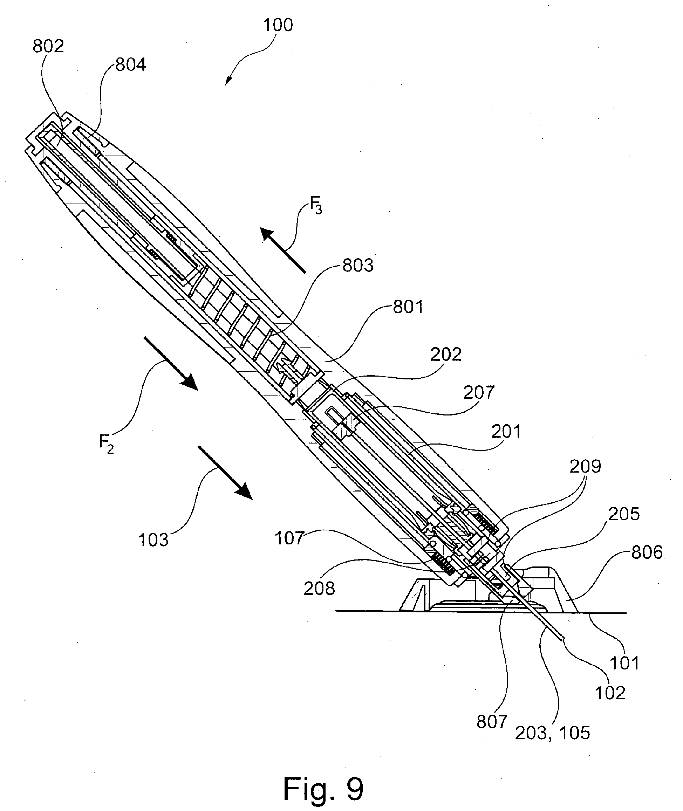

Ein Verriegelungselement 805 (z.B. ein Verriegelungshaken) verriegelt den Kolben 202 in einer Initialposition, in welche die zweite Feder 803 vorgespannt ist. Ein Auslöseelement 802 (z.B. ein Druckknopf) ist mit dem Verriegelungselement 805 derart gekoppelt, dass bei Betätigen des Auslöseelements 802, beispielsweise durch einen Benutzer, das Verriegelungselement 805 den Kolben 202 freigibt und der Kolben 202 mit dem Nadelhalter 204 mittels der zweiten Federkraft F2 entlang der Einstechrichtung 103 vortreibbar ist (vgl. Fig. 8 mit Fig. 9). Eine dritte Feder 804 kann mit einer dritten Federkraft F3 das Auslöseelement 802 zurück in eine Startposition verschieben.

Nach dem Erreichen der subkutanen Endposition des Katheters 205 (Fig. 9 bis Fig. 11) wird z.B. mittels des Einsatzes der Rückholfeder 107 selbsttätig die Setznadel 203 von dem Katheter 205 entkoppelt und entgegen der Einstechrichtung 103 aus dem Körper herausgezogen. Im Zusammenspiel mit der zweiten Feder 803 wird somit ein selbsttätiger Setz-Mechanismus geschaffen. Der Benutzer muss lediglich beispielsweise das Auslöseelement 802 betätigen, um die Setzhilfe 100 zu aktivieren. Nach dem Betätigen des Auslöseelements 802 verschiebt sich der Kolben 202 mit dem Nadelhalter 204 und mit der Setznadel 203 zusammen mit dem Katheter 205 bis zur subkutanen Endposition. Anschließend wird der Nadelhalter 204 aus dem Körper mittels der Rückholfeder 107 herausgezogen, ohne dass der Benutzer weitere Schritte unternehmen muss.

Der Grundkörper 201 ist ferner austauschbar an einem Auflagekörper 806 derart befestigt, dass der Grundkörper 201 mit einem vorbestimmten Einstechwinkel zwischen Setznadel 203 und Hautoberfläche fixiert ist. Der Auflagekörper 806 kann beispielsweise mittels einer Klebeverbindung (z.B. mittels eines Pflasters) auf der Haut 101 befestigt werden. Der Auflagekörper 806 weist z.B. Koppelelemente auf, welche den Grundkörper 201 austauschbar an den Auflagekörper 806 koppeln. Beispielsweise kann der Grundkörper 201 mittels eines Bajonettverschlusses an dem Auflagekörper 806 befestigt werden.

Der Auflagekörper 806 weist ferner ein Befestigungselement bzw. einen Katheterhalter 807 auf, welcher eingerichtet ist, bei Vorliegen der subkutanen Endposition des Katheters 205 den Katheter 205 an den Auflagekörper 806 zu befestigen.

Der Katheterhalter 807 bildet mit dem Katheter 205 beispielsweise eine kraftschlüssige oder eine formschlüssige Verbindung (z.B. eine SchnappHaken-Verbindung) aus. Beispielsweise kann der Katheterhalter 807 mit dem Katheter 205 einen Bajonettverschluss ausbilden.

Der Katheterhalter 807 ist beispielsweise schwenkbar in dem Auflagekörper 806 angeordnet und in einer gewünschten Schwenkposition selektiv verriegelbar. Der Benutzer kann somit eine gewünschte Schwenkposition einstellen, so dass der Katheterhalter 807 den Katheter 205 bzw. die Auslösevorrichtung 200 und die Setzvorrichtung 800 derart hält, dass ein gewünschter erster oder zweiter Einstechwinkel α, β einstellbar ist.

Der Einstechwinkel α, β definiert einen Winkel zwischen der Hautoberfläche und der Längsachse der Setznadel 203. Der Einstechwinkel α, β liegt beispielsweise zwischen 0° und 90°. Je flacher der Winkel, desto näher liegt die subkutane Endposition an der Hauptoberfläche. Je steiler der Winkel, zum Beispiel bei 90°, desto tiefer befindet sich die subkutane Endposition im Inneren der Haut bzw. des Gewebes.

Durch die Wahlmöglichkeit des Einstechwinkels α, β kann der Benutzer den Einstechwinkel α, β an die Dicke seiner/ihrer subkutanen Fettschicht anpassen, sodass bei gegebener Katheter- und Sensordrahtlänge die proximalen Enden von Katheter 205 und Sensordraht 210 im Fettgewebe zu liegen kommen und nicht in das darunter liegende Muskelgewebe hinein reichen. Das Austausch-Set bzw. die Austauschvorrichtung 200 kann dadurch vorteilhaft mit einer einzigen Katheter- und Sensordrahtlänge realisiert werden.

Nach Erreichen der subkutanen Endposition ist der Katheter 205 von dem Grundkörper 201 entkoppelt. Entsprechend kann der Grundkörper 201 von dem Auflagekörper 806 gelöst werden, wobei der Katheter 205 weiter an den Auflagekörper 806 fixiert bleibt und von dem Grundkörper 201 getrennt werden kann.

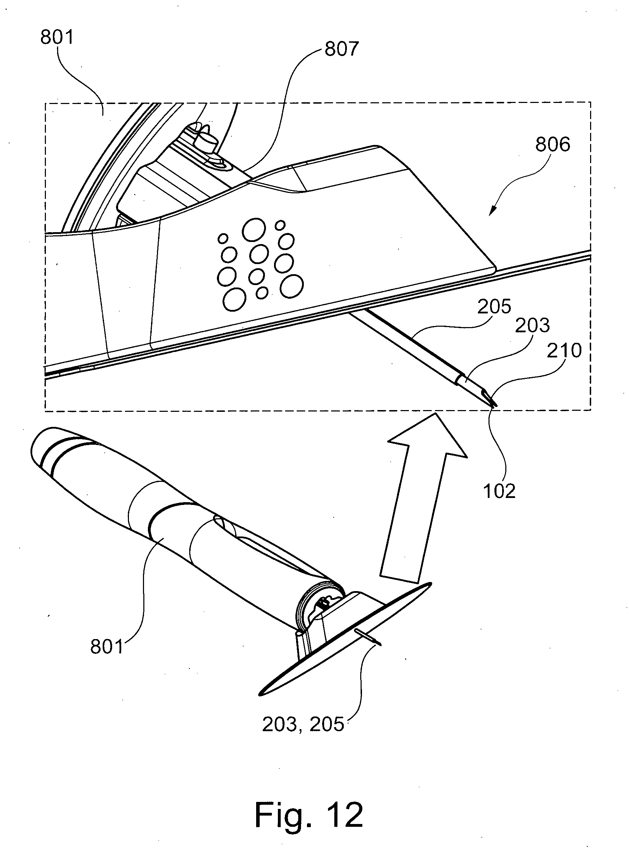

**Fig. 12** zeigt eine perspektivische Darstellung der Setzhilfe 100 aus Fig. 8 bis Fig. 11.

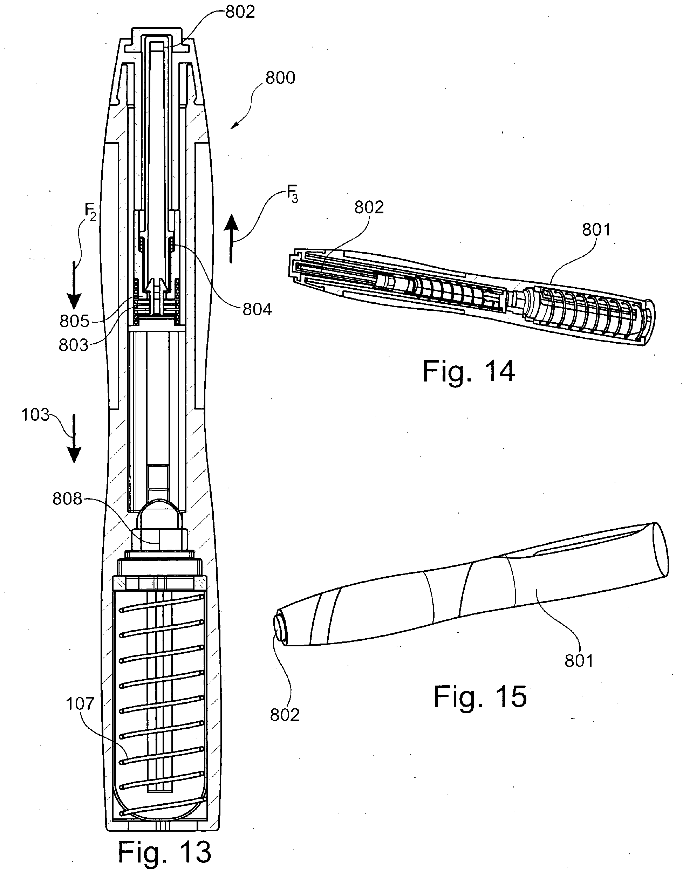

**Fig. 13** bis **Fig. 15** zeigen schematische Darstellungen des Gehäusekörpers 801. Die Setzhilfe 100 weist eine hohlzylindrische Form auf. In einem Kopfbereich des Gehäusekörpers 801 ist das Auslöseelement 802 angeordnet. Das Auslöseelement 802 ist hohlzylindrisch ausgebildet, wobei das Auslöseelement 802 im Inneren des Gehäusekörpers 801 eine Öffnung aufweist. Bei Verschieben des Auslöseelements 802 in Einstechrichtung 103 stülpt sich das Auslöseelement 802 über die Verriegelungselemente 805. Die Verriegelungselemente 805 werden dadurch zusammengedrückt und die zweite Feder 803 freigegeben. Dadurch kann die zweite Feder 803 die zweite Federkraft F2 auf den Kolben 202 aufbringen und diesen in Einstechrichtung 103 verschieben. In Fig. 13 ist ferner ein Rückholring 808 dargestellt, in welchen der Kolben 202 austauschbar befestigbar ist. Nach Betätigen des Auslöseelements 802 drückt die dritte Feder 804 mit der dritten Federkraft F3 das Auslöseelement 802 erneut in eine Startposition.

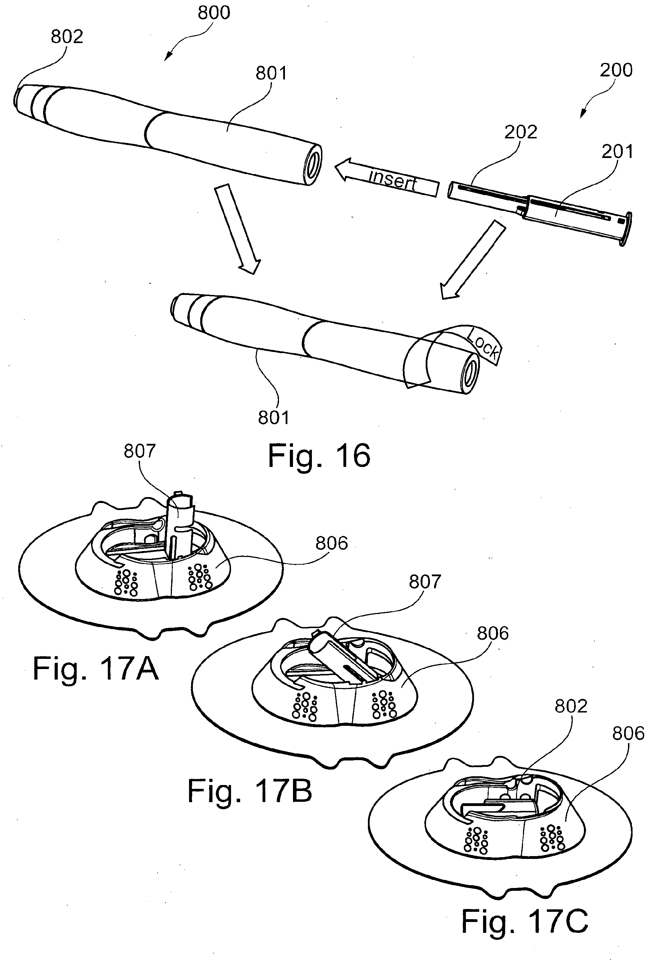

**Fig. 16** zeigt einen Befestigungsvorgang der Setzvorrichtung 800 mit der Austauschvorrichtung 200, d.h. mit dem Grundkörper 201 und dem Kolben 202. Beispielsweise kann die Setzvorrichtung 800 mit dem Grundkörper 201 der Austauschvorrichtung 200 mittels eines Bajonettverschluss verbunden werden. Somit kann der Kolben 202 zusammen mit dem Grundkörper 201 in das Gehäuse 801 hineingeschoben werden und mittels einer Viertel- oder Halbdrehung befestigt werden. Entsprechend kann nach dem Setzen des Sensordrahtes und der Kolben 202 und der Grundkörper 201 mittels einer Viertel- oder einer Halbdrehung von der Setzvorrichtung 800 entfernt werden.

Beispielsweise kann der Grundkörper 201 mittels eines Bajonettverschluss mit dem Auflagekörper 806 verbunden werden. Nach dem Setzen des Katheters 205 und des Sensordrahtes 210 bleiben diese in dem Auflagekörper 806 fixiert. Die Setzvorrichtung 800 zusammen der Austauschvorrichtung 200 (insbesondere mit dem Grundkörper 201 und dem Kolben 202) können von dem Auflagekörper 806 gelöst werden. Der Katheterhalter 807 kann beispielsweise schwenkbar an dem Auflagekörper 806 befestigt sein, um den Einstechwinkel einzustellen.

Nachdem der Katheter 205 in die subkutane Endposition gebracht worden ist und an dem Auflagekörper 806 befestigt ist, kann der Katheter 205 für die weitere Verwendung eingesetzt werden. Beispielsweise kann anschließend an den Sensordraht 210 die Sensor-Ausleseeinheit oder eine Pumpeneinheit zur Infusion eines Medikaments an den Katheter 205 befestigt werden.

Die Sensor-Ausleseeinheit ist an dem Auflagekörper 806 derart austauschbar befestigbar, dass die Sensor-Ausleseeinheit mit dem Sensordraht 210, welcher sich in dem Katheter 205 befindet, zum Signalaustausch gekoppelt ist. Entsprechend kann eine Pumpeneinheit an dem Auflagekörper 806 derart austauschbar befestigbar sein, dass die Pumpeneinheit mit dem Katheter 205 gekoppelt ist, so dass ein Medikament von der Pumpeneinheit in den Katheter 205 infundiert und somit zu der subkutanen Endposition befördert werden kann.

**Fig. 17A** bis **Fig. 17C** zeigen schematische Darstellungen eines Auflagekörpers 806 mit einem verstellbaren Katheterhalter 807. Der Katheterhalter 807 ist schwenkbar an dem Auflagekörper befestigt, und kann in einer gewünschten Schwenkposition verriegelt bzw. fixiert werden. Der Katheter 205 ist in dem Katheterhalter 807 fixierbar. Somit kann mittels des Katheterhalters 807 ein gewünschter Einstechwinkel α, β eingestellt werden.

Ferner zeigt Fig. 17C, dass der Katheterhalter 807 in den Auflagekörper 806 einklappbar ist und somit von Umgebungseinflüssen geschützt ist. In dem Katheterhalter 807 kann der Katheter 205 isoliert von der Setzhilfe 100 angeordnet sein. So kann zunächst der Katheter 205 mit Hilfe der Setzhilfe 100 gesetzt werden und anschließend die Setzhilfe 100 von dem Katheter 205 gelöst werden, so dass der Katheter 205 in dem Katheterhalter 807 vorliegt. Wenn kein Medikament zugeführt wird oder keine Messung mittels des Sensordrahts 210 vorgenommen wird, kann der Katheter 205 zusammen mit dem Katheterhalter 807 in die inaktive geschützte Stellung, wie in Fig. 17C dargestellt, geschwenkt werden.

Ergänzend ist darauf hinzuweisen, dass "umfassend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden ist, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

**Bezugszeichenliste:**

| | | | |
|---|---|---|---|
| 100 | Setzhilfe | 701 | erster Spalt |
| 101 | Haut/Gewebe | 702 | zweiter Spalt |
| 102 | Spitze | | |
| 103 | Einstechrichtung | 800 | Setzvorrichtung |
| 104 | Befestigungsbereich | 801 | Gehäusekörper |
| 105 | subkutaner Bereich des Katheters | 802 | Auslöseelement |
| | | 803 | zweite Feder |
| 106 | erste Feder | 804 | dritte Feder |
| 107 | Rückholfeder | 805 | Verriegelungselement |
| 108 | Hautöffnung | 806 | Auflagekörper |
| 109 | Gewebe | 807 | Katheterhalter |
| | | 808 | Rückholring |
| 200 | Austauschvorrichtung | | |
| 201 | Grundkörper | F1 | erste Federkraft |
| 202 | Kolben | F2 | zweite Federkraft |
| 203 | Setznadel | F3 | dritte Federkraft |
| 204 | Nadelhalter | Fr | Rückholfederkraft |
| 205 | Katheter | | |
| 206 | Niederhaltestab | α, β | Einstechwinkel |
| 207 | Stempel | | |
| 208 | Klemmkörper | | |
| 209 | Sensorkontakt | | |
| 210 | Sensordraht | | |
| 211 | Kerbe | | |
| 212 | Schnapphaken | | |
| 213 | Keilfläche | | |

## Patentansprüche

1. Setzhilfe (100) zum Setzen eines Katheters (205) und eines Sensordrahtes (210) in einen Körper, wobei die Setzhilfe (100) aufweist
eine Setzvorrichtung (800),
eine Austauschvorrichtung (200) mit einem Grundkörper (201), einer Setznadel (203), einem Katheter (205) und einem Sensordraht (210),
wobei die Austauschvorrichtung (200) in der Setzvorrichtung (800) auswechselbar und austauschbar angeordnet ist,
wobei die Setznadel (203) an den Grundkörper (201) gekoppelt ist,
wobei die Setznadel (203) und der Katheter (205) derart zueinander angeordnet sind,
dass die Setznadel (203) innerhalb des Katheters (205) angeordnet ist, und
dass eine Spitze (102) der Setznadel (203) von einem proximalen Ende des Katheters (205) entlang einer Einstechrichtung (103) herausragt, so dass bei dem Setzen des Katheters (205) entlang der Einstechrichtung (103) die Spitze (102) der Setznadel (203) die Haut (101) penetriert, um eine Hautöffnung (108) zu generieren, durch welche der Katheter (205) bis zu einer subkutanen Endposition nachführbar ist,
wobei der Sensordraht (210) mit der Setznadel (203) derart gekoppelt ist,
dass bei dem Setzen des Katheters (205) entlang der Einstechrichtung (103) der Sensordraht (210) in der Setznadel (203) angeordnet ist, und
dass bei Erreichen der subkutanen Endposition des Katheters (205) die Setznadel (203) entgegen der Einstechrichtung (103) aus dem Katheter (205) herausziehbar ist und der Sensordraht (210) in dem Katheter (205) verbleibt.

2. Setzhilfe (100) gemäß Anspruch 1,
wobei die Austauschvorrichtung (200) ferner einen Kolben (202) und einen Nadelhalter (204), an welchem die Setznadel (203) befestigt ist, aufweist, wobei der Kolben (202) in dem Grundkörper (201) verschiebbar angeordnet ist,
wobei der Nadelhalter (204) mit der Setznadel (203) entlang der Einstechrichtung (103) verschiebbar an dem Grundkörper (201) angeordnet ist,
wobei der Nadelhalter (204) mit dem Katheter (205) derart gekoppelt ist, dass bei dem Setzen des Katheters (205) der Nadelhalter (204) den Katheter (205) entlang der Einstechrichtung (103) vortreibt, und
wobei der Kolben (202) mit dem Nadelhalter (204) derart gekoppelt ist, dass mittels des Kolbens (202) der Nadelhalter (204) entlang der Einstechrichtung (103) vortreibbar ist.

3. Setzhilfe (100) gemäß Anspruch 1 oder 2,
wobei in einem Befestigungsbereich (104) des Katheters (205) ein Klemmkörper (208) angeordnet ist,
wobei der Klemmkörper (208) die kraftschlüssige Verbindung mit der Setznadel (203) ausbildet.

4. Setzhilfe (100) gemäß Anspruch 2 oder 3, ferner aufweisend
eine erste Feder (106), welche zwischen dem Grundkörper (201) und dem Nadelhalter (204) derart angeordnet ist, dass eine erste Federkraft (F1) der ersten Feder (106) entlang der Einstechrichtung (103) wirkt, um bei dem Setzen des Katheters (205) den Nadelhalter (204) entlang der Einstechrichtung (103) relativ zu dem Grundkörper (201) vorzutreiben.

5. Setzhilfe (100) gemäß einem der Ansprüche 2 bis 4,
wobei der Kolben (202) teleskopartig in den Grundkörper (201) ein- und ausfahrbar ausgebildet ist.

6. Setzhilfe (100) gemäß einem der Ansprüche 2 bis 5,
wobei der Kolben (202) mit dem Nadelhalter (204) ferner derart lösbar gekoppelt ist, dass bei Erreichen der subkutanen Endposition der Nadelhalter (204) von dem Kolben (202) entkoppelbar ist und der Nadelhalter (204) relativ zu dem Kolben (202) entgegen der Einstechrichtung (103) verschiebbar ist.

7. Setzhilfe (100) gemäß einem der Ansprüche 2 bis 6, ferner aufweisend
eine zweite Feder (803), welche zwischen der Setzvorrichtung (800) und der Austauschvorrichtung (200) derart angeordnet ist, dass eine zweite Federkraft (F2) der zweiten Feder (803) entlang der Einstechrichtung (103) wirkt, um bei dem Setzen des Katheters (205) den Nadelhalter (204) entlang der Einstechrichtung (103) vorzutreiben,
wobei die zweite Feder (803) derart eingerichtet ist, dass bei einem Verschieben des Nadelhalters (204) entgegen der Einstechrichtung (103) die zweite Feder (803) vorspannbar ist,
wobei die Setzvorrichtung (800) ein Verriegelungselement (805) und ein Auslöseelement (802) aufweist,
wobei das Verriegelungselement (805) den Nadelhalter (204) in einer Initialposition verriegelt, in welcher die zweite Feder (803) vorgespannt ist, wobei das Auslöseelement (802) mit dem Verriegelungselement (805) derart gekoppelt ist, dass bei Betätigen des Auslöseelements (802) das Verriegelungselement (805) den Nadelhalter (204) freigibt und der Nadelhalter (204) mittels der zweiten Federkraft entlang der Einstechrichtung (103) vortreibbar ist.

8. Setzhilfe (100) gemäß Anspruch 7,
wobei der Kolben (202) in der Setzvorrichtung (800) verschiebbar angeordnet ist,
wobei der Kolben (202) einerseits mit der zweiten Feder (803) und andererseits mit dem Nadelhalter (204) derart gekoppelt ist, dass der Kolben (202) und der Nadelhalter (204) mittels der zweiten Federkraft entlang der Einstechrichtung (103) vortreibbar sind.

9. Setzhilfe (100) gemäß Anspruch 7, ferner aufweisend
eine dritte Feder (804), welche zwischen der Setzvorrichtung (800) und der Austauschvorrichtung (200) derart angeordnet ist, dass eine dritte Federkraft (F3) der dritten Feder (804) entgegen der Einstechrichtung (103) wirkt, um das Auslöseelement (802) und das Verriegelungselement (805) zurück in die Initialposition verschiebt.

10. Setzhilfe (100) gemäß einem der Ansprüche 2 bis 9, ferner aufweisend
eine Rückholfeder (107), welche zwischen der Setzvorrichtung (800) und dem Nadelhalter (204) derart angeordnet ist, dass eine Rückholkraft der Rückholfeder (107) entgegen der Einstechrichtung (103) wirkt, um nach dem Setzen des Katheters (205) den Nadelhalter (204) entgegen der Einstechrichtung (103) vorzutreiben,
wobei die Rückholfeder (107) derart eingerichtet ist, dass bei einem Verschieben des Nadelhalters (204) entlang der Einstechrichtung (103) die Rückholfeder (107) vorspannbar ist.

11. Setzhilfe (100) gemäß einem der Ansprüche 2 bis 10, ferner aufweisend
einen Niederhaltestab (206), welcher in der Setznadel (203) verschieblich angeordnet ist,
wobei der Niederhaltestab (206) ausgebildet ist, den Sensordraht (210) kraftschlüssig gegen die Setznadel (203) zu drücken,
wobei der Niederhaltestab (206) ferner derart entlang der Einstechrichtung (103) verschiebbar an dem Grundkörper (201) angeordnet ist,
dass bei dem Setzen des Katheters (205) entlang der Einstechrichtung (103) die Setznadel (203), der Sensordraht (210) und der Niederhaltestab (206) bis zu der subkutanen Endposition in der Haut (101) oder des Gewebes (109) zusammen vortreibbar sind, und
dass bei Erreichen der subkutanen Endposition des Katheters (205) der Niederhaltestab (206) entgegen der Einstechrichtung (103) aus dem Katheter (205) herausziehbar ist,
wobei der Niederhaltestab (206) ferner derart an dem Grundkörper (201) angeordnet ist, dass bei Erreichen der subkutanen Endposition des Katheters (205) der Niederhaltestab (206) aus dem Katheter (205) herausziehbar ist, nachdem die Setznadel (203) zumindest teilweise aus dem Katheter (205) herausgezogen ist,
wobei der Niederhaltestab (206) einen Stempel (207) aufweist, welcher mittels einer kraftschlüssigen Verbindung an den Kolben (202) befestigt ist,
wobei der Stempel (207) ferner derart in dem Kolben (202) angeordnet ist, dass in einer vorbestimmten Position im Kolben (202) der Nadelhalter (204) entgegen der Einstechrichtung (103) gegen den Stempel (207) derart drückt, dass die kraftschlüssige Verbindung zwischen den Kolben (202) und dem Stempel (207) überwindbar ist und der Stempel (207) mit dem Niederhaltestab (206) mittels des Nadelhalters (204) entgegen der Einstechrichtung (103) relativ zu dem Kolben (202) verschiebbar ist.

12. Setzhilfe (100) gemäß einem der Ansprüche 1 bis 11, ferner aufweisend
einen Auflagekörper (806), welcher auf der Haut (101) befestigbar ist, wobei der Grundkörper (201) und/oder die Setzvorrichtung (800) austauschbar an dem Auflagekörper (806) derart befestigbar ist, dass der Grundkörper (201) mit einem vorbestimmten Einstechwinkel (α, β) zwischen Setznadel (203) und Hautoberfläche fixierbar ist,
wobei der Auflagekörper (806) ein Befestigungselement aufweist, welches eingerichtet ist, bei Vorliegen der subkutanen Endposition des Katheters (205) den Katheter (205) an den Auflagekörper (806) zu befestigen.

13. Setzhilfe (100) gemäß Anspruch 12, ferner aufweisend
eine Sensor-Ausleseeinheit, welche an den Auflagekörper (806) derart austauschbar befestigbar ist, dass die Sensor-Ausleseeinheit mit dem Sensordraht (210) gekoppelt ist.

14. Setzhilfe (100) gemäß Anspruch 12 oder 13, ferner aufweisend
eine Pumpeneinheit zur Injektion eines Medikaments,
wobei die Pumpeneinheit an den Auflagekörper (806) derart austauschbar befestigbar ist, dass die Pumpeneinheit mit dem Katheter (205) gekoppelt ist.

15. Verfahren zum Setzen eines Katheters (205) in einen Körper mit einer Setzhilfe (100) gemäß einem der Ansprüche 1 bis 14, wobei das Verfahren aufweist
Anordnen der Austauschvorrichtung (200) in der Setzvorrichtung (800),
Auflegen der Setzhilfe (100) auf einer Haut (101) des Körpers,
Setzen des Katheters (205) mittels der Setzhilfe (100), und
Entfernen und Austauschen der Austauschvorrichtung (200) aus der Setzvorrichtung (800).

## Claims

1. Placement aid (100) for placing a catheter (205) and a sensor wire (210) into a body, wherein the placement aid (100) comprises
a placement device (800),
a replacement device (200) with a main body (201, a placement needle (203), a catheter (205 and a sensor wire (210),
wherein the replacement device (200) is arranged in the placement device (800) in an interchangeable and replaceable manner,
wherein the placement needle (203) is coupled to the main body (201),
wherein the placement needle (203) and the catheter (205) are arranged with regard to each other in such a way
that the placement needle (203) is arranged within the catheter (205) and
that a tip (102) of the placement needle (203) protrudes from a proximal end of the catheter (205) along a puncturing direction (103) so that upon placement of the catheter (205) along the puncturing direction (103) the tip (102) of the placement needle (203) penetrates the skin (101) in order to produce a skin opening (108) through which the catheter (205) can be guided up to a subcutaneous end position,
wherein the sensor wire (210) is coupled to the placement needle (203) in such a way
that upon placement of the catheter (205) along the puncturing direction (103) the sensor wire (210) is arranged in the placement needle (203) and
that upon reaching the subcutaneous end position of the catheter (205) the placement needle (203) can be removed from the catheter (205) against the puncturing direction (103) and the sensor wire (21) remains in the catheter (205).

2. Placement aid (100) according to claim 1,
wherein the replacement device (200) further comprises a piston (202) and a needle holder (204), to which the placement needle (203) is attached, wherein the piston (200) is arranged in the main body (201) in a displaceable manner, wherein the needle holder (203) with the placement needle (203) is arranged at the main body (201) so as to be displaceable along the puncturing direction (103),
wherein the needle holder (204) is coupled to the catheter (205) in such a way that upon placement of the catheter (205) the needle holder (204) advances the catheter (205) along the puncturing direction (103), and
wherein the piston (202) is coupled to the needle holder (204) in such a way that the needle holder (204) can be advanced along the puncturing direction (103) by means of the piston (202).

3. Placement aid (100) according to claim 1 or 2,
wherein a clamping element (208) is arranged in a fastening section (104) of the catheter (205),
wherein the clamping element (208) forms the force-fitting connection with the placement needle (203).

4. Placement aid (100) according to claim 2 or 3, further comprising
a first spring (106) which is arranged between the main body (201) and the needle holder (204) in such a way that a first spring force (F1) of the first spring (106) acts along the puncturing direction (103) in order to advance the needle holder (204) along the puncturing direction (103) relative the basic body (201) upon the placement of the catheter (205).

5. Placement aid (100) according to any one of claims 2 to 4, wherein
the piston (202) is designed to be introduced into and retracted from the main body (201) in a telescopic manner.

6. Placement aid (100) according to any one of claims 2 to 5, wherein
the piston (202) is detachably coupled to the needle holder (204) in such a way that upon reaching the subcutaneous end position the needle holder (204) can be decoupled from the piston (202) and the needle holder (204) can be moved relative to the piston against the puncturing direction (103).

7. Placement aid (100) according to any one of claims 2 to 6, further comprising
a second spring (803) which is arranged between the placement device (800) and the replacement device (200) in such a way that a second spring force (F2) of the second spring (803) acts along the puncturing direction (103) in order to advance the needle holder (204) along the puncturing direction (103) upon placement of the catheter (205),
wherein the second spring (803) is set up in such a way that, when the needle holder (204) is moved against the puncturing direction (103), the second spring (803) can be pre-tensioned,
wherein the placement device (800) comprises a locking element (805) and trigger element (802),
wherein the locking element (805) locks the needle holder (204) in an initial position, in which the second spring (803) is pre-tensioned,
wherein the trigger element (802) is coupled to the locking element (805) in such a way that when operating the trigger element (802) the locking element (805) releases the needle holder (204) and the needle holder (204) can be advanced along the puncturing direction (103) by means of the second spring force.

8. Placement aid (100) according to claim 7,
wherein the piston (202) is arranged in the placement device (800) in a displaceable manner,
wherein the piston (202) is coupled on the one hand to the second spring (803) and on the other hand to the needle holder (204) in such a way that the piston (202) and the needle holder (204) can be advanced along the puncturing direction (103) by means of the second spring force.

9. Placement aid (100) according to claim 7, further comprising
a third spring (804) which is arranged between the placement device (800) and the replacement device (200) in such a way that a third spring force (F3) of the third spring (804) acts against the puncturing direction (103) in order to displace the trigger element (802) and the locking element (805) back into the initial position.

10. Placement aid (100) according to any one of claims 2 to 9, further comprising
a return spring (107) which is arranged between the placement device (800) and the needle holder (204) in such a way that return force of the return spring (107) acts against the puncturing direction (103) in order to advance the needle holder (204) against the puncturing direction (103) after placement of the catheter (205),
wherein the return spring (107) is set up in such a way that when the needle holder (204) moves along the puncturing direction (103) the return spring (107) can be pre-tensioned.

11. Placement aid (100) according to any one of claims 2 to 10, further comprising
a holding-down rod (206) which is arranged in the placement needle (203) in a displaceable manner,
wherein the holding-down rod (206) is designed to frictionally press the sensor wire (210) against the placement needle (203),
wherein the holding-down rod (206) is further arranged at the main body (201) in a movable manner along the puncturing direction (103)
such that upon placement of the catheter (205) along the puncturing direction (103) the placement needle (203), the sensor wire (210) and the holding-down rod (206) can jointly be advanced up to the subcutaneous end position in the skin (101) or the tissue (109) and
such that upon reaching the subcutaneous end position of the catheter (205) the holding-down rod can be withdrawn from the catheter (205) against the puncturing direction (103),
wherein the holding-down rod (206) is further arranged at the main body (201) in such a way that upon reaching the subcutaneous end position of the catheter (205) the holding-down rod (206) can be withdrawn from the catheter (205) after the placement needle (203) has at least partially been pulled out of the catheter (205),
wherein the holding-down rod (206) comprises plunger (207) which is attached to the piston (202) by mean of a force-fitting connection,
wherein the plunger (207) is further arranged in the piston (202) in such a way that at a predetermined position in the piston (202) the needle holder (204) presses against the plunger (207) against the puncturing direction (103) in such a way that the force-fitting connection between the piston (202) and the plunger (207) can be overcome and the plunger (207) with the holding-down rod (206) can be moved by means of the needle holder (204) against the puncturing direction (103) relative to the piston (202).

12. Placement aid (100) according to any one of claims 1 to 11, further comprising
a support element (806) which can be attached to the skin (101),
wherein the main body (201) and/or the placement device (800) can be attached to the support element (806) in a replaceable manner in such a way that the main body (201) can be affixed with a predetermined puncture angle (α, β) between the placement needle (203) and skin surface,
wherein the support element (806) comprises a fastening element which is designed to fasten the catheter (205) to the support element (806) when the catheter (205) is in the subcutaneous end position.

13. Placement aid (100) according to claim 12, further comprising
a sensor read-out unit, which can be attached in a replaceable manner to the support element (806) in such a way that the sensor read-out unit is coupled to the sensor wire (210).

14. Placement aid (100) according to any one of claims 12 or 13, further comprising
a pump unit for injecting a medication, wherein the pump unit can be attached at the support element (806) in a replaceable manner in such a way that the pump unit is coupled to the catheter (205).

15. Method of placing a catheter (205) in to a body with a placement aid (100) according to any one of claims 1 to 14, wherein the method comprises
arranging the replacement device (200) in the placement device (800),
applying the placement aid (100) on a skin (101) of the body,
placing the catheter (205) by means of the placement aid (100) and
removing and replacing the replacement device (200) from the placement device (800).

## Revendications

1. Auxiliaire de placement (100) pour placer un cathéter (205) et un fil de capteur (210) dans un corps, **caractérisé en ce que** l'auxiliaire de placement (100) présente
un dispositif de placement (800),
un dispositif d'échange (200) avec un corps de base (201), une aiguille de placement (203), un cathéter (205) et un fil de capteur (210),
le dispositif d'échange (200) étant disposé dans le dispositif de placement (800) de manière remplaçable et échangeable,
l'aiguille de placement (203) étant couplée au corps de base (201),
l'aiguille de placement (203) et le cathéter (205) étant disposés l'un par rapport à l'autre de telle sorte
que l'aiguille de placement (203) est disposée à l'intérieur du cathéter (205), et
qu'une pointe (102) de l'aiguille de placement (203) dépasse d'une extrémité proximale du cathéter (205) le long d'un sens de piqûre (103), de sorte que lors du placement du cathéter (205) le long du sens de piqûre (103), la pointe (102) de l'aiguille de placement (203) pénètre dans la peau (101) pour générer un orifice cutané (108), par lequel le cathéter (205) peut être guidé jusqu'à une position finale sous-cutanée,
le fil de capteur (210) étant couplé avec l'aiguille de placement (203) de telle sorte
que lors du placement du cathéter (205) le long du sens de piqûre (103), le fil de capteur (210) est disposé dans l'aiguille de placement (203), et
que lorsque la position finale sous-cutanée du cathéter (205) est atteinte, l'aiguille de placement (203) peut être retirée du cathéter (205) dans le sens contraire au sens de piqûre (103) et le fil de capteur (210) reste dans le cathéter (205).

2. Auxiliaire de placement (100) selon la revendication 1,
**caractérisé en ce que** le dispositif d'échange (200) présente en outre un piston (202) et un porte-aiguille (204), sur lequel l'aiguille de placement (203) est fixée, le piston (202) étant disposé de manière déplaçable dans le corps de base (201),
**en ce que** le porte-aiguille (204) est disposé avec l'aiguille de placement (203) au niveau du corps de base (201) de manière déplaçable le long du sens de piqûre (103),
**en ce que** le porte-aiguille (204) est couplé avec le cathéter (205) de telle sorte que lors du placement du cathéter (205), le porte-aiguille (204) fait avancer le cathéter (205) le long du sens de piqûre (103), et
**en ce que** le piston (202) est couplé avec le porte-aiguille (204) de telle sorte que le porte-aiguille (204) peut être avancé le long du sens de piqûre (103) au moyen du piston (202).

3. Auxiliaire de placement (100) selon la revendication 1 ou 2,
**caractérisé en ce qu'**un corps de serrage (208) est disposé dans une zone de fixation (104) du cathéter (205),
le corps de serrage (208) réalisant la liaison par force avec l'aiguille de placement (203).

4. Auxiliaire de placement (100) selon la revendication 2 ou 3, présentant en outre
un premier ressort (106), lequel est disposé entre le corps de base (201) et le porte-aiguille (204) de telle sorte qu'une première force de ressort (F1) du premier ressort (106) agit le long du sens de piqûre (103) pour faire avancer le porte-aiguille (204) le long du sens de piqûre (103) par rapport au corps de base (201) lors du placement du cathéter (205).

5. Auxiliaire de placement (100) selon l'une quelconque des revendications 2 à 4,
**caractérisé en ce que** le piston (202) est réalisé de façon rétractable et déployable à la manière d'un télescope dans le corps de base (201).

6. Auxiliaire de placement (100) selon l'une quelconque des revendications 2 à 5,
**caractérisé en ce que** le piston (202) est couplé en outre de manière amovible avec le porte-aiguille (204) de telle sorte que lorsque la position finale sous-cutanée est atteinte, le porte-aiguille (204) peut être découplé du piston (202) et le porte-aiguille (204) peut être déplacé par rapport au piston (202) dans le sens contraire au sens de piqûre (103).

7. Auxiliaire de placement (100) selon l'une quelconque des revendications 2 à 6, présentant en outre
un deuxième ressort (803), lequel est disposé entre le dispositif de placement (800) et le dispositif d'échange (200) de telle sorte qu'une deuxième force de ressort (F2) du deuxième ressort (803) agit le long du sens de piqûre (103) pour faire avancer le porte-aiguille (204) le long du sens de piqûre (103) lors du placement du cathéter (205),
**caractérisé en ce que** le deuxième ressort (803) est agencé de telle sorte que lors d'un déplacement du porte-aiguille (204) dans le sens contraire au sens de piqûre (103), le deuxième ressort (803) peut être précontraint,
le dispositif de placement (800) présentant un élément de verrouillage (805) et un élément de déclenchement (802),
l'élément de verrouillage (805) verrouillant le porte-aiguille (204) dans une position initiale, dans laquelle le deuxième ressort (803) est précontraint,
l'élément de déclenchement (802) étant couplé avec l'élément de verrouillage (805) de telle sorte que lors de l'actionnement de l'élément de déclenchement (802), l'élément de verrouillage (805) libère le porte-aiguille (204) et le porte-aiguille (204) peut être avancé le long du sens de piqûre (103) au moyen de la deuxième force de ressort.

8. Auxiliaire de placement (100) selon la revendication 7,
**caractérisé en ce que** le piston (202) est disposé de manière déplaçable dans le dispositif de placement (800),
le piston (202) étant couplé d'un côté avec le deuxième ressort (803) et d'un autre côté avec le porte-aiguille (204) de telle sorte que le piston (202) et le porte-aiguille (204) peuvent être avancés le long du sens de piqûre (103) au moyen de la deuxième force de ressort.

9. Auxiliaire de placement (100) selon la revendication 7, présentant en outre
un troisième ressort (804), lequel est disposé entre le dispositif de placement (800) et le dispositif d'échange (200) de telle sorte qu'une troisième force de ressort (F3) du troisième ressort (804) agit dans le sens contraire au sens de piqûre (103) pour replacer l'élément de déclenchement (802) et l'élément de verrouillage (805) dans la position initiale.

10. Auxiliaire de placement (100) selon l'une quelconque des revendications 2 à 9, présentant en outre
un ressort de rappel (107), lequel est disposé entre le dispositif de placement (800) et le porte-aiguille (204) de telle sorte qu'une force de rappel du ressort de rappel (107) agit dans le sens contraire au sens de piqûre (103) pour faire avancer le porte-aiguille (204) dans le sens contraire au sens de piqûre (103) après le placement du cathéter (205),
**caractérisé en ce que** le ressort de rappel (107) est agencé de telle sorte que lors d'un déplacement du porte-aiguille (204) le long du sens de piqûre (103), le ressort de rappel (107) peut être précontraint.

11. Auxiliaire de placement (100) selon l'une quelconque des revendications 2 à 10, présentant en outre
une tige serre-flan (206), laquelle est disposée de manière mobile dans l'aiguille de placement (203),
**caractérisé en ce que** la tige serre-flan (206) est réalisée pour pousser le fil de capteur (210) par force contre l'aiguille de placement (203),
la tige serre-flan (206) étant disposée en outre au niveau du corps de base (201) de manière déplaçable le long du sens de piqûre (103) de telle sorte
que lors du placement du cathéter (205) le long du sens de piqûre (103), l'aiguille de placement (203), le fil de capteur (210) et la tige serre-flan (206) peuvent être avancés ensemble jusqu'à la position finale sous-cutanée dans la peau (101) ou le tissu (109), et
que lorsque la position finale sous-cutanée du cathéter (205) est atteinte, la tige serre-flan (206) peut être retirée du cathéter (205) dans le sens contraire au sens de piqûre (103),
la tige serre-flan (206) étant disposée en outre au niveau du corps de base (201) de telle sorte que lorsque la position finale sous-cutanée du cathéter (205) est atteinte, la tige serre-flan (206) peut être retirée du cathéter (205), après que l'aiguille de placement (203) a été retirée au moins partiellement du cathéter (205),
la tige serre-flan (206) présentant un poinçon (207), lequel est fixé sur le piston (202) au moyen d'une liaison par force,
le poinçon (207) étant disposé en outre dans le piston (202) de telle sorte que dans une position prédéterminée dans le piston (202), le porte-aiguille (204) pousse dans le sens contraire au sens de piqûre (103) contre le poinçon (207) de telle sorte que la liaison par force entre le piston (202) et le poinçon (207) peut être surmontée et le poinçon (207) peut être déplacé avec la tige serre-flan (206) dans le sens contraire au sens de piqûre (103) par rapport au piston (202) au moyen du porte-aiguille (204).

12. Auxiliaire de placement (100) selon l'une quelconque des revendications 1 à 11, présentant en outre
un corps d'appui (806), lequel peut être fixé sur la peau (101),
**caractérisé en ce que** le corps de base (201) et/ou le dispositif de placement (800) peut être fixé au corps d'appui (806) de manière échangeable de telle sorte que le corps de base (201) peut être fixé avec un angle de piqûre prédéterminé (α, β) entre l'aiguille de placement (203) et la surface cutanée,
le corps d'appui (806) présentant un élément de fixation, lequel est agencé pour fixer le cathéter (205) au corps d'appui (806) en cas de position finale sous-cutanée du cathéter (205).

13. Auxiliaire de placement (100) selon la revendication 12, présentant en outre
une unité de lecture de capteur, laquelle peut être fixée au corps d'appui (806) de manière échangeable de telle sorte que l'unité de lecture de capteur est couplée avec le fil de capteur (210).

14. Auxiliaire de placement (100) selon la revendication 12 ou 13, présentant en outre
une unité de pompage pour l'injection d'un médicament,
**caractérisé en ce que** l'unité de pompage peut être fixée au corps d'appui (806) de manière échangeable de telle sorte que l'unité de pompage est couplée avec le cathéter (205).

15. Procédé de placement d'un cathéter (205) dans un corps avec un auxiliaire de placement (100) selon l'une quelconque des revendications 1 à 14, **caractérisé en ce que** le procédé présente
l'agencement du dispositif d'échange (200) dans le dispositif de placement (800),
la pose de l'auxiliaire de placement (100) sur la peau (101) du corps,
le placement du cathéter (205) au moyen de l'auxiliaire de placement (100), et
le retrait et l'échange du dispositif d'échange (200) du dispositif de placement (800).
